(19) Europäisches Patentamt
European Patent Office
Office européen des brevets

(11) **EP 4 153 142 B1**

(12) **EUROPEAN PATENT SPECIFICATION**

(45) Date of publication and mention
of the grant of the patent:
**22.07.2026 Bulletin 2026/30**

(21) Application number: **21729601.1**

(22) Date of filing: **19.05.2021**

(51) International Patent Classification (IPC):
*A61K 9/50* (2006.01)    *A61K 9/10* (2006.01)
*A61K 9/06* (2006.01)    *A61K 38/16* (2006.01)
*A61K 38/17* (2006.01)    *A61K 38/38* (2006.01)
*A61K 38/40* (2006.01)    *A61K 47/36* (2006.01)
*A61P 1/02* (2006.01)

(52) Cooperative Patent Classification (CPC):
**A61K 9/1075; A61K 38/168; A61K 38/40;
A61K 47/36; A61P 1/02**

(86) International application number:
**PCT/GB2021/051212**

(87) International publication number:
**WO 2021/234386 (25.11.2021 Gazette 2021/47)**

(54) **FORMULATION COMPRISING A PROTEINACEOUS MICROGEL**

FORMULIERUNG MIT EINEM PROTEINÖSEN MIKROGEL

FORMULATION COMPRENANT UN MICROGEL PROTÉIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.05.2020 GB 202007546**

(43) Date of publication of application:
**29.03.2023 Bulletin 2023/13**

(73) Proprietor: **University of Leeds
Leeds LS2 9JT (GB)**

(72) Inventors:
• **SARKAR, Anwesha**
**Leeds Leeds West Yorkshire LS2 9JT (GB)**
• **ANDABLO REYES, Efren, Alberto**
**Leeds Leeds West Yorkshire LS2 9JT (GB)**
• **HU, Jing**
**Leeds Leeds West Yorkshire LS2 9JT (GB)**
• **PABOIS, Olivia**
**Leeds Leeds West Yorkshire LS2 9JT (GB)**

(74) Representative: **HGF
HGF Limited
4th Floor, 1 City Square
Leeds LS1 2ES (GB)**

(56) References cited:
**EP-A1- 2 489 779**    **CN-A- 108 578 357**
**US-A1- 2009 191 276**

• **CALAHORRA A A: "Pickering emulsion-based
encapsulation strategies for delivery of
curcumin", 1 March 2020 (2020-03-01), pages 1 -
263, XP093310136, Retrieved from the Internet
<URL:https://core.ac.uk/download/pdf/
327079097.pdf>**
• **JIN BEI ET AL: "Self-Assembled Modified Soy
Protein/Dextran Nanogel Induced by
Ultrasonication as a Delivery Vehicle for
Riboflavin", MOLECULES, vol. 21, no. 3, 15
March 2016 (2016-03-15), DE, pages 282,
XP055833555, ISSN: 1433-1373, DOI: 10.3390/
molecules21030282**
• **RAVANFAR RAHELEH ET AL: "Optimization of
microcapsules shell structure to preserve labile
compounds: A comparison between
microfluidics and conventional homogenization
method", FOOD CHEMISTRY, ELSEVIER LTD,
NL, vol. 241, 7 September 2017 (2017-09-07),
pages 460 - 467, XP085196680, ISSN: 0308-8146,
DOI: 10.1016/J.FOODCHEM.2017.09.023**

EP 4 153 142 B1

- OLIVARES M L ET AL: "Soft lubrication characteristics of microparticulated whey proteins used as fat replacers in dairy systems", JOURNAL OF FOOD ENGINEERING, BARKING ESSEX, GB, vol. 245, 13 October 2018 (2018-10-13), pages 157 - 165, XP085533806, ISSN: 0260-8774, DOI: 10.1016/ J.JFOODENG.2018.10.015
- SARKAR ANWESHA ET AL: "Lubrication of soft oral surfaces", CURRENT OPINION IN COLLOID & INTERFACE SCIENCE, vol. 39, 1 February 2019 (2019-02-01), GB, pages 61 - 75, XP055833653, ISSN: 1359-0294, Retrieved from the Internet <URL:https://www.sciencedirect.com/science/ article/pii/S1359029418301006/pdfft? md5=2eeab82d9b9d076f41cac8708d0343dd& pid=1-s2.0-S1359029418301006-main.pdf> DOI: 10.1016/j.cocis.2019.01.008
- HU JING ET AL: "Synergistic Microgel-Reinforced Hydrogels as High-Performance Lubricants", ACS MACRO LETTERS, vol. 9, no. 12, 16 November 2020 (2020-11-16), pages 1726 - 1731, XP055833501, ISSN: 2161-1653, Retrieved from the Internet <URL:http://pubs.acs.org/doi/ pdf/10.1021/acsmacrolett.0c00689> DOI: 10.1021/acsmacrolett.0c00689

Remarks:

The file contains technical information submitted after the application was filed and not included in this specification

**Description**

This invention relates to a formulation comprising a proteinaceous microgel and one or more polysaccharide-based nanofibrils. The invention also relates to methods for preparing such formulations. The invention also contemplates the use of such formulations as a lubricant food additive, and such formulations for use in the treatment of a disease or condition selected from dry mouth, xerostomia, or dysphagia.

**BACKGROUND**

Due to the growing cases of lubrication-failure related oral diseases, such as xerostomia (dry mouth), the development of new biocompatible lubricants with high performance under oral conditions has become an important research subject.

Xerostomia (the subjective sensation of dry mouth) is a common symptom with estimated prevalence of roughly 20% in the general population and up to 50% in the elderly [Furness S, Worthington HV, Bryan G, Birchenough S, McMillan R. Interventions for the management of dry mouth: topical therapies. Cochrane Database of Systematic Reviews. 2011; Hopcraft M, Tan C. Xerostomia: an update for clinicians. 2010;55:238-44].

The complaint of dry mouth can be related to objective symptoms of hyposalivation, such as: reduced salivary flow, change in the composition of saliva, or dry oral tissues, but it is also reported by people with normal salivary gland function [Villa A, Abati S. Risk factors and symptoms associated with xerostomia: a cross-sectional study. 2011;56:290-5]. There are several causes of xerostomia. A key clinical cause of dry mouth conditions is head and neck radiation therapy for cancers, which causes degeneration of salivary glands tissue, leading to reduction of saliva secretion depending on the radiation dose and treatment area [Łysik D, Niemirowicz-Laskowska K, Bucki R, Tokajuk G, Mystkowska J. Artificial Saliva: Challenges and Future Perspectives for the Treatment of Xerostomia. 2019;20:3199]. Other possible causes can be diseases including salivary gland diseases and disorders, chronic inflammatory autoimmune diseases like Sjögren's syndrome, endocrine diseases like diabetes, neurologic diseases and disorders, psychogenic diseases and conditions like anxiety and nervousness, and infections like HIV/ AIDS [Furness S, Worthington HV, Bryan G, Birchenough S, McMillan R. Interventions for the management of dry mouth: topical therapies. Cochrane Database of Systematic Reviews. 2011; Łysik D, Niemirowicz-Laskowska K, Bucki R, Tokajuk G, Mystkowska J. Artificial Saliva: Challenges and Future Perspectives for the Treatment of Xerostomia. 2019;20:3199]. In addition, polypharmacy such as consuming multiple drugs at the same time including antihypertensives, opiates, antidepressants, antipsychotics, bronchodilators, proton pump inhibitors, antineoplastics, antihistamines, diuretics, and others can also induce dry mouth conditions [Porter SR, Scully C, Hegarty AM. An update of the etiology and management of xerostomia. Oral Surgery, Oral Medicine, Oral Pathology, Oral Radiology, and Endodontology. 2004;97:28-46; Thelin W, Brennan M, Lockhart P, Singh M, Fox P, Papas A, et al. The oral mucosa as a therapeutic target for xerostomia. 2008;14:683-9].

Different therapies for xerostomia have been developed according to the diagnosis of the severity and causes [Närhi TO, Meurman JH, Ainamo A. Xerostomia and Hyposalivation. Drugs & Aging. 1999;15:103-16]. Typical treatment for xerostomia can involve the stimulation of the secretion of saliva, either pharmaceutically or by mechanical stimulation, and/or can involve symptomatic treatment like application of oral mucosal lubricants and/or salivary substitutes for the palliation of the symptoms [Łysik D, Niemirowicz-Laskowska K, Bucki R, Tokajuk G, Mystkowska J. Artificial Saliva: Challenges and Future Perspectives for the Treatment of Xerostomia. 2019;20:3199; Han P, Suarez-Durall P, Mulligan R. Dry mouth: A critical topic for older adult patients. Journal of Prosthodontic Research. 2015;59:6-19]. The symptomatic therapies generally aim at moistening the oral mucosa [Narhi TO, Meurman JH, Ainamo A. Xerostomia and hyposalivation: causes, consequences and treatment in the elderly. Drugs Aging. 1999;15:103-16]. Frequent fluid intake like water and glycerine or other biopolymers can be useful for periodic relief for dry mouth, but often the relief is short-lived. This is because most available therapies look at thickeners that just tend to increase the viscosity of water rather than focusing on lubrication properties, which are crucial aspects for salivary performance [Łysik D, Niemirowicz-Laskowska K, Bucki R, Tokajuk G, Mystkowska J. Artificial Saliva: Challenges and Future Perspectives for the Treatment of Xerostomia. 2019;20:3199]. Therefore, in the domain of salivary substitutes or oral moisturizers in the form of rinses, spray, gel, toothpastes or lozenges, there is a clear technology gap on systems that provide the necessary lubrication properties required for adequate treatment of xerostomia.

Recent studies have shown that microgels have potential to act as bio-lubricants [Andablo-Reyes E, Yerani D, Fu M, Liamas E, Connell S, Torres O, et al. Microgels as viscosity modifiers influence lubrication performance of continuum. Soft Matter. 2019;15:9614-24; Sarkar A, Kanti F, Gulotta A, Murray BS, Zhang S. Aqueous lubrication, structure and rheological properties of whey protein microgel particles. Langmuir. 2017;33:14699-708; Torres O, Andablo-Reyes E, Murray BS, Sarkar A. Emulsion microgel particles as high-performance bio-lubricants. ACS Applied Materials & Interfaces. 2018;10:26893-905] due to their capacity to reduce friction in soft contacts due to aqueous lubrication mechanism. However, the lubrication performance shown by microgels in relevant oral conditions is still poor in comparison to human saliva, which is an excellent bio-lubricant [Xu F, Liamas E, Bryant M, Adedeji AF, Andablo-Reyes E, Castronovo M, et al. A self-assembled binary protein model explains high-performance salivary lubrication from macro to nanoscale. Advanced Materials Interfaces. 2020;7:1901549].

Therefore, there is a need in the art for the provision of alternative or better treatments for xerostomia.

Swallowing disorder is one of the common results of dry mouth, which can significantly decrease the quality of life. Therefore, various compositions in the form of beverages or solid foods, such as chewing gum, candy and chocolate have been developed to facilitate mastication and deglutition of the food product [JP2018064512 "Solid food product easy in mastication and deglutition"; JP2016063832 "Packed beverage"]. For example, previous patents containing either salivary secretion promoting component or polysaccharide thickener have been reported. However, salivary secretion promoting components are effective only if there is remaining salivary function. Also, the efficiency of polysaccharide thickeners commonly used for such purpose have limited lubrication properties [Han, P., P. Suarez-Durall, and R. Mulligan, Dry mouth: a critical topic for older adult patients. J Prosthodont Res, 2015. 59(1): p. 6-19].

CN108578357 discloses a protein-polysaccharide self-assembly nano-gel with a core-shell structure, a preparation method of the gel and an application of the gel. The nano-gel takes natural polymer proteins and polysaccharides as raw materials, and the raw materials are simply self-assembled to form the nano-gel with the core-shell structure. The nano-gel takes a protein as an inner core and takes a polysaccharide as a shell. The preparation of the nano-gel mainly adopts the electrostatic interaction between the protein and the polysaccharide and gelatination properties of the protein. The nano-gel is suitable for carriers of various hydrophobic micro-molecule anti-cancer drugs and can wrap micromolecule drugs in the forming process of the gel, and the nano-gel loading the anti-cancer drugs is acquired.

Olivares M L at al "Soft lubrication characteristics of microparticulated whey proteins used as fat replacers in dairy systems", Journal of Food Engineering, vol. 245, 13 October 2018, pages 157-165 discloses the fat mimicking mechanism of microparticulated whey proteins (MWP) in milk-based systems using rheological and tribological techniques. Flow curves and friction measurements in a soft contact of skim milk-MPW dispersions (SM-MPW) and skim milk-dairy fat emulsions (SM-DF) at different concentrations (3, 6, 9, 12, 15, 18 and 20% w/w) and temperatures (25 °C and 37 °C) were carried out and compared. Friction coefficient curves of SM-MPW dispersions as a function of the product of entrainment speed and viscosity collapsed into a single master curve in the mixed and elastohydrodynamic (EHL) regimes when the high shear viscosity values as obtained through Carreau-Yasuda model were used. This suggests that the dispersions as a whole entrained in the contact. However, in the case of SM-DF emulsions, a very good collapse is obtained if only a SM is assumed to pass through the contact. Simulations of friction coefficient within the EHL region showed a reasonably good continuity of the experimental data for SM-MPW dispersions and SM-DF emulsions. Finally, it was observed that friction levels attained with MPW proteins and DF at typical speeds involved in oral processing were comparable, hence demonstrating the capability of SM-MPW proteins dispersions to imitate DF in milk-based systems from a lubrication point of view.

Anwesha Sarkar et al: "Lubrication of soft oral surfaces", Current Opinion in Colloid & Interfacial Science, vol. 39, 1 February 2019, pages 61-75 discloses a description of soft oral surfaces, comparing them with the recent approaches that have been used to study oral lubrication using *in vitro* to *ex vivo* setups. Specifically, lubrication behaviours of saliva and soft microgels are discussed highlighting instances of hydration lubrication.

Andrea Araiza Calahorra, The University of Leeds: "Pickering emulsion-based encapsulation strategies for delivery of curcumin", 1 March 2020, pages 1-263 discloses the design of complex particulate Pickering interfaces using two types of interactions (i.e. electrostatic and covalent) between whey protein and polysaccharides (dextran sulphate or dextran). Hence, three different Pickering emulsions; whey protein isolate nanogel particle-stabilized ($E_{WPN}$), dextran sulphate coated-whey protein isolate nanogel particle-stabilized ($D_XS-E_{WPN}$) and whey protein isolate-dextran conjugated (or Maillard) microgel particle-stabilized ($E_{WPDXM}$) Pickering emulsions were created as delivery vehicles for curcumin.

Therefore, there is a need in the art for the provision of alternative or better compositions that facilitate mastication and deglutition of food products.

## BRIEF SUMMARY OF THE DISCLOSURE

According to a first aspect, there is provided a method for preparing a formulation as claimed in claim 1.

According to a second aspect, there is provided a formulation of claim 7.

According to a third aspect, there is provided a use of a formulation as a lubricant food additive or a formulation for use in the treatment of a disease or condition selected from: dry mouth, xerostomia, or dysphagia of claim 8.

The present invention relates to aqueous formulations having bio-lubrication properties. Preferably, the formulations have improved bio-lubrication properties compared to commercially available bio-lubricants and human saliva.

The present invention relates to formulations having a low friction coefficient. Preferably, the formulations have a lower friction coefficient compared to commercially available bio-lubricants and human saliva.

The present invention relates to formulations having a low viscosity. Preferably, the formulations have lower viscosity compared to commercially available bio-lubricants.

**BRIEF DESCRIPTION OF THE DRAWINGS**

Embodiments of the invention are further described hereinafter with reference to the accompanying drawings. In accordance with the invention are only those embodiments falling within the scope of the claims. Any other embodiments are disclosed for illustrative purposes. In the drawings:

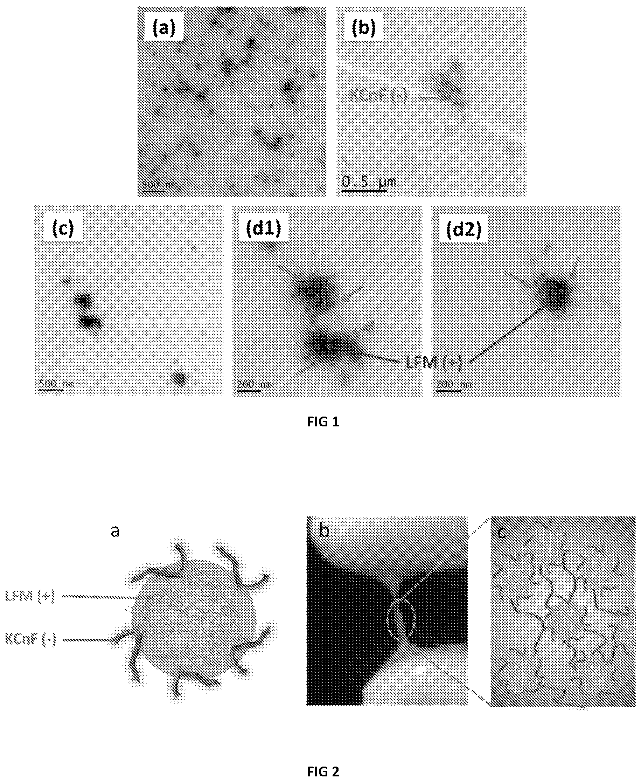

Figure 1 shows TEM images of examples of the formulation of the present invention made following Example 1. (a) TEM image of lactoferrin microgel (LFM) with scale bar of 500 nm. (b) TEM image of κ-carrageenan nanofibrils (KCnF) with scale bar of 500 nm. (c) TEM image of LFM partially covered by KCnF with scale bar of 500 nm, and (d1)(d2) with scale bar of 200 nm.

Figure 2 shows examples of the formulation of the present invention made following Example 1. (a) Schematic diagram of LFM coated with KCnF. (b) Photograph of a formulation of the present invention shown under uniaxial extensional flow exerted manually between fingertips (thumb and forefinger), such behaviour is also shown by real human saliva. (c) Schematic of the structure created by the formulation containing a plurality of LFM microgel particles connected by interaction of adjacent KCnF nanofibrils.

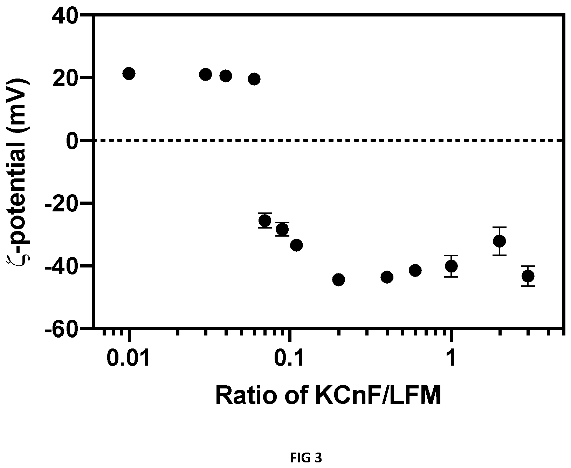

Figure 3 shows the $\zeta$-potential of the formulation as a function of different weight ratios of KCnF:LFM (from 0.01:1 to 3:1 w/w) made following Example 1. (The weight ratio of the one or more polysaccharide-based nanofibrils to the proteinaceous microgel of the formulations of the present invention is from about 0.1:1 to about 10:1.) As the relative concentration of KCnF increases, a steep inversion in the sign of $\zeta$-potential is observed.

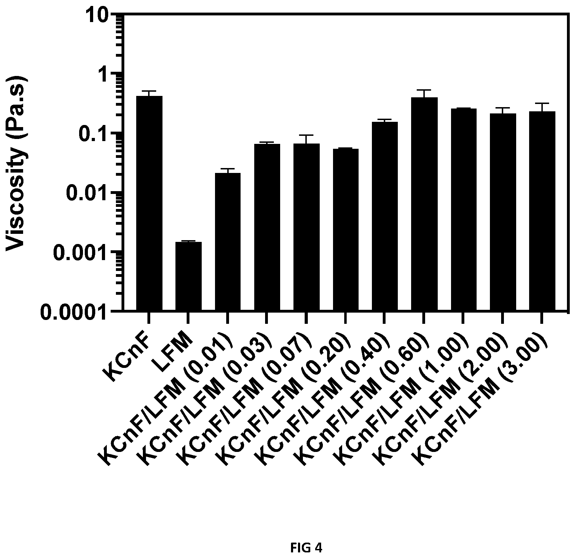

Figure 4 shows the shear viscosity at the orally relevant shear rate of LFM, KCnF, and exemplary formulations of the invention made following Example 1 (comprising KCnF and LFM in a ratio of 0.01:1 to 3:1 w/w). (The weight ratio of the one or more polysaccharide-based nanofibrils to the proteinaceous microgel of the formulations of the present invention is from about 0.1:1 to about 10:1.)

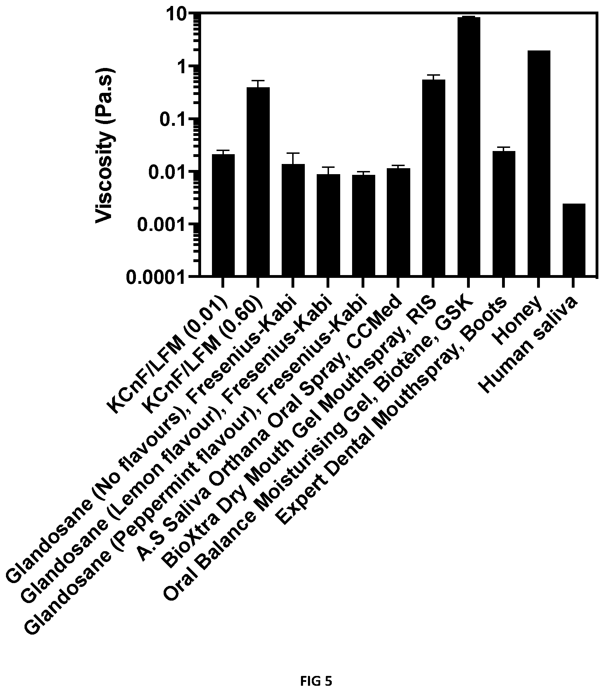

Figure 5 shows the shear viscosity at the orally relevant shear rate of exemplary formulations of the invention made following Example 1 (comprising KCnF and LFM in a ratio of 0.01:1 and 0.60:1 w/w), honey, human saliva, and various commercially available saliva replacement gel and spray products. (The weight ratio of the one or more polysaccharide-based nanofibrils to the proteinaceous microgel of the formulations of the present invention is from about 0.1:1 to about 10:1.)

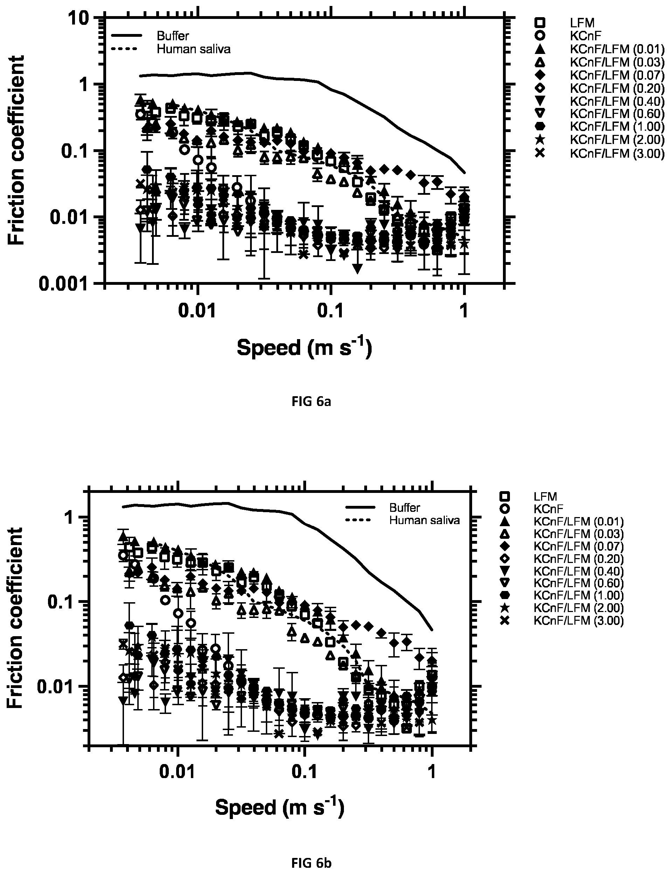

Figure 6a and Figure 6b shows the friction coefficients as a function of speed obtained for exemplary formulations of the present invention made following Example 1 (comprising KCnF and LFM in a ratio of 0.01:1 to 3:1 w/w), and compares these values with those obtained for LFM, KCnF, human saliva and buffer. (The weight ratio of the one or more polysaccharide-based nanofibrils to the proteinaceous microgel of the formulations of the present invention is from about 0.1:1 to about 10:1.) The data provided in Figure 6b is the same data as is that in Figure 6a. The Figures differ in that the y-axis has been altered from 0.001-10 (in Figure 6a) to 0.002-2 (in Figure 6b).

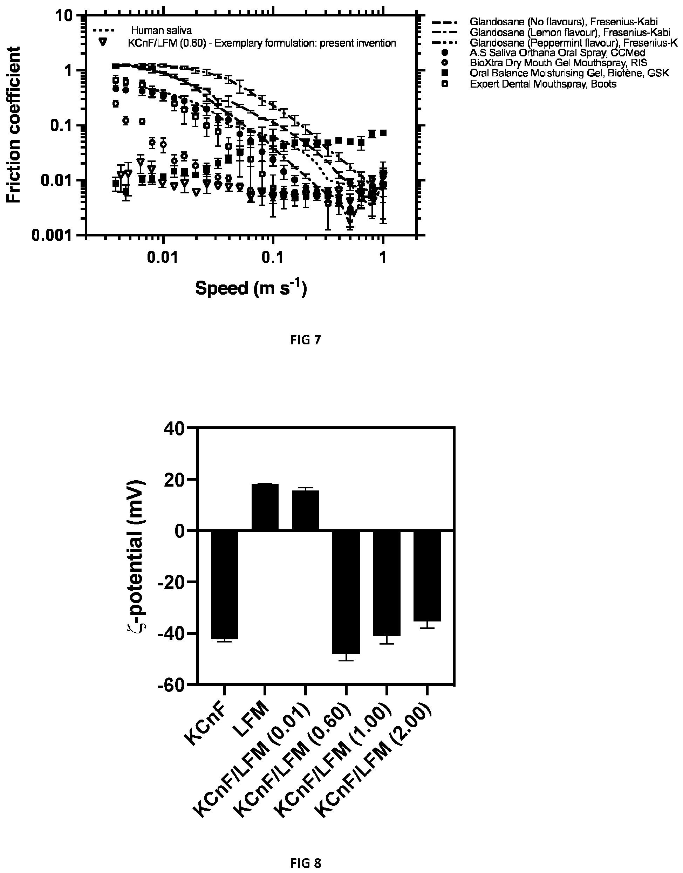

Figure 7 shows the friction coefficients as a function of speed obtained for one exemplary formulation of the present invention made following Example 1 (comprising KCnF and LFM in a ratio of 0.60:1 w/w), and compares these values with those obtained for human saliva and various commercially available saliva replacement gel and spray products.

Figure 8 shows the $\zeta$-potential of the formulation as a function of different weight ratios of KCnF:LFM (from 0.01:1 to 2:1 w/w) made following Example 2, and compares these values with those obtained for KCnF and LFM. (The weight ratio of the one or more polysaccharide-based nanofibrils to the proteinaceous microgel of the formulations of the present invention is from about 0.1:1 to about 10:1.) As the relative concentration of KCnF increases, a steep inversion in the sign of $\zeta$-potential is observed.

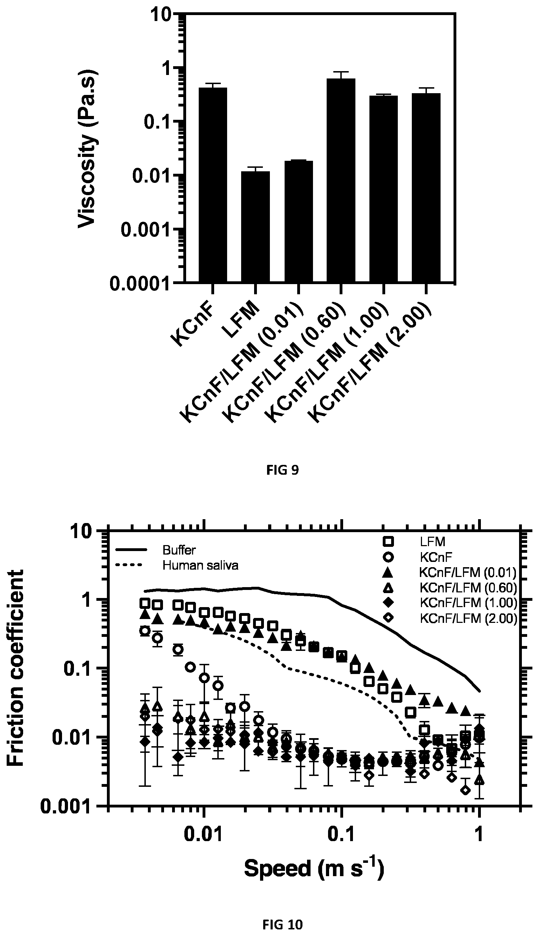

Figure 9 shows the shear viscosity at the orally relevant shear rate of LFM, KCnF, and exemplary formulations of the invention made following Example 2 (comprising KCnF and LFM in a ratio of 0.01:1 to 2:1 w/w). (The weight ratio of the one or more polysaccharide-based nanofibrils to the proteinaceous microgel of the formulations of the present invention is from about 0.1:1 to about 10:1.)

Figure 10 shows the friction coefficients as a function of speed obtained for exemplary formulations of the present invention made following Example 2 (comprising KCnF and LFM in a ratio of 0.01:1 to 2:1 w/w), and compares these values with those obtained for LFM, KCnF, human saliva and buffer. (The weight ratio of the one or more polysaccharide-based nanofibrils to the proteinaceous microgel of the formulations of the present invention is from about 0.1:1 to about 10:1.)

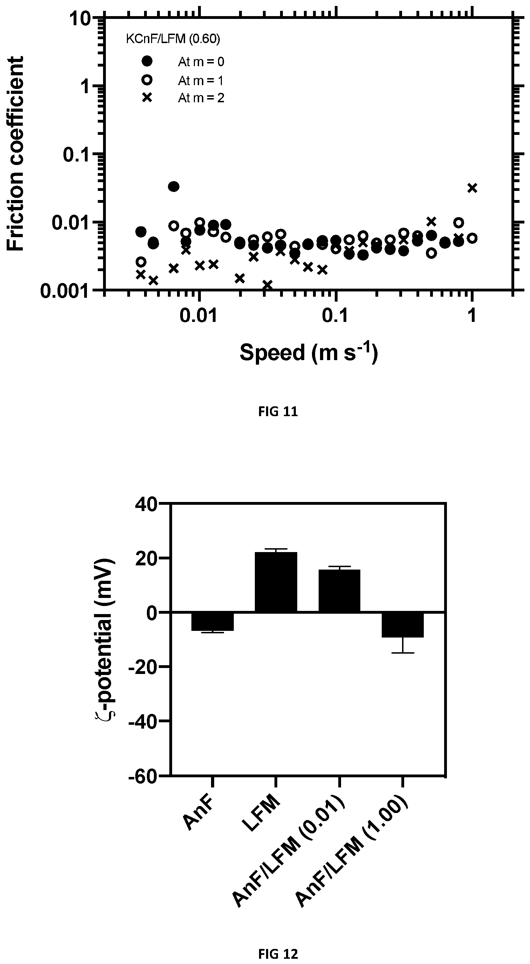

Figure 11 shows the friction coefficients as a function of speed obtained for exemplary formulations of the present invention made following Example 2 (comprising KCnF and LFM in a ratio of 0.60:1 w/w) at 0 month, 1 month and 2 months storage.

Figure 12 shows the $\zeta$-potential of the formulation as a function of different weight ratios of AnF:LFM (from 0.01:1 to 1:1 w/w) made following Example 3, and compares these values with those obtained for AnF and LFM. (The weight ratio of the one or more polysaccharide-based nanofibrils to the proteinaceous microgel of the formulations of the present invention is from about 0.1:1 to about 10:1.)

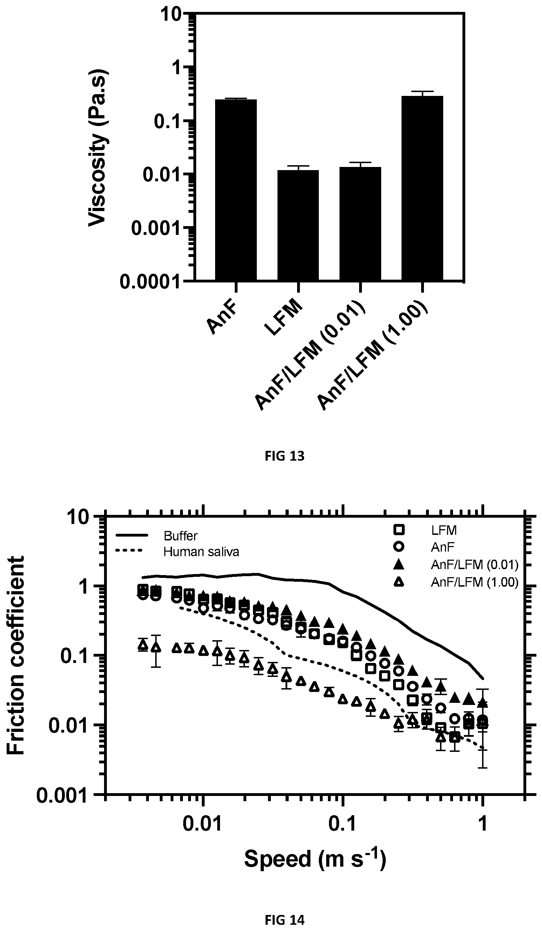

Figure 13 shows the shear viscosity at the orally relevant shear rate of LFM, AnF, and exemplary formulations of the invention made following Example 3 (comprising AnF and LFM in a ratio of 0.01:1 to 1:1 w/w). (The weight ratio of the one or more polysaccharide-based nanofibrils to the proteinaceous microgel of the formulations of the present invention is from about 0.1:1 to about 10:1.)

Figure 14 shows the friction coefficients as a function of speed obtained for exemplary formulations of the present invention made following Example 3 (comprising AnF and LFM in a ratio of 0.01:1 to 1:1 w/w), and compares these values with those obtained for LFM, AnF, human saliva and buffer. (The weight ratio of the one or more polysaccharide-based nanofibrils to the proteinaceous microgel of the formulations of the present invention is from about 0.1:1 to about 10:1.)

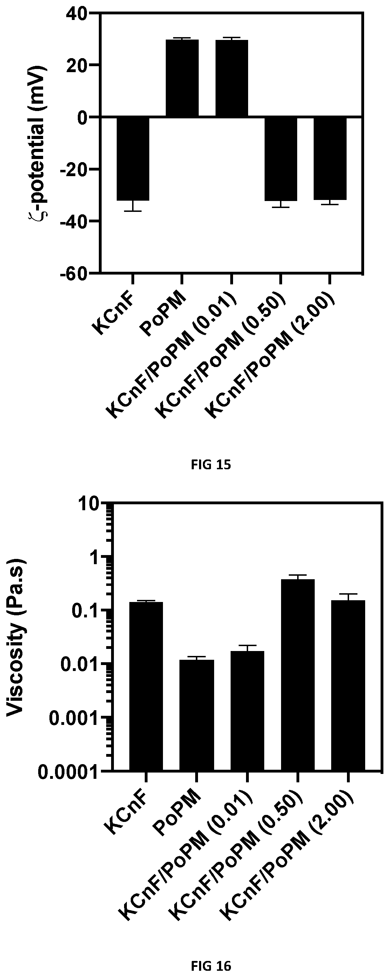

Figure 15 shows the $\zeta$-potential of the formulation as a function of different weight ratios of KCnF: PoPM (from 0.01:1 to 2:1 w/w) made following Example 4, and compares these values with those obtained for KCnF and PoPM. (The weight ratio of the one or more polysaccharide-based nanofibrils to the proteinaceous microgel of the formulations of the present invention is from about 0.1:1 to about 10:1.)

Figure 16 shows the shear viscosity at the orally relevant shear rate of PoPM, KCnF, and exemplary formulations of the invention made following Example 4 (comprising KCnF and PoPM in a ratio of 0.01:1 to 2:1 w/w). (The weight ratio of the one or more polysaccharide-based nanofibrils to the proteinaceous microgel of the formulations of the present invention is from about 0.1:1 to about 10:1.)

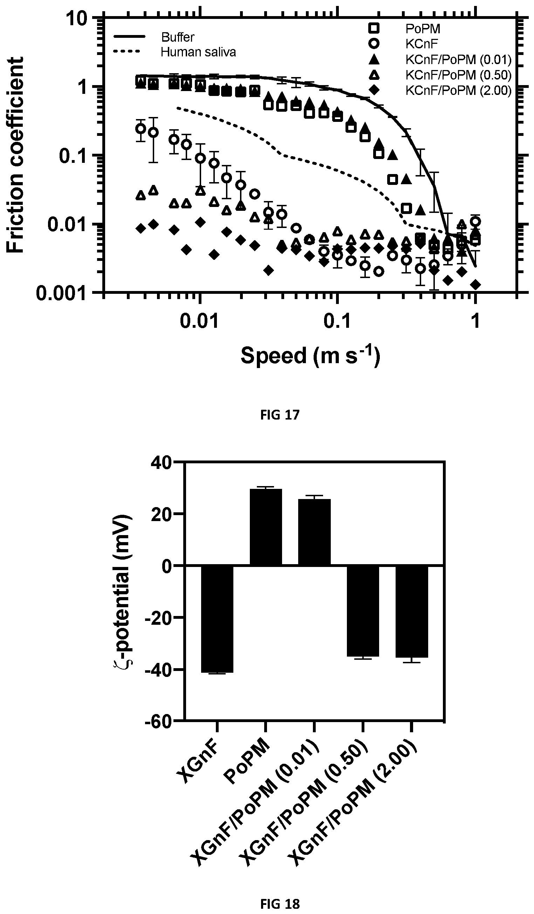

Figure 17 shows the friction coefficients as a function of speed obtained for exemplary formulations of the present invention made following Example 4 (comprising KCnF and PoPM in a ratio of 0.01:1 to 2:1 w/w), and compares these values with those obtained for PoPM, KCnF, human saliva and buffer. (The weight ratio of the one or more polysaccharide-based nanofibrils to the proteinaceous microgel of the formulations of the present invention is from about 0.1:1 to about 10:1.)

Figure 18 shows the $\zeta$-potential of the formulation as a function of different weight ratios of XGnF: PoPM (from 0.01:1 to 2:1 w/w) made following Example 5, and compares these values with those obtained for XGnF and PoPM. (The weight ratio of the one or more polysaccharide-based nanofibrils to the proteinaceous microgel of the formulations of the present invention is from about 0.1:1 to about 10:1.)

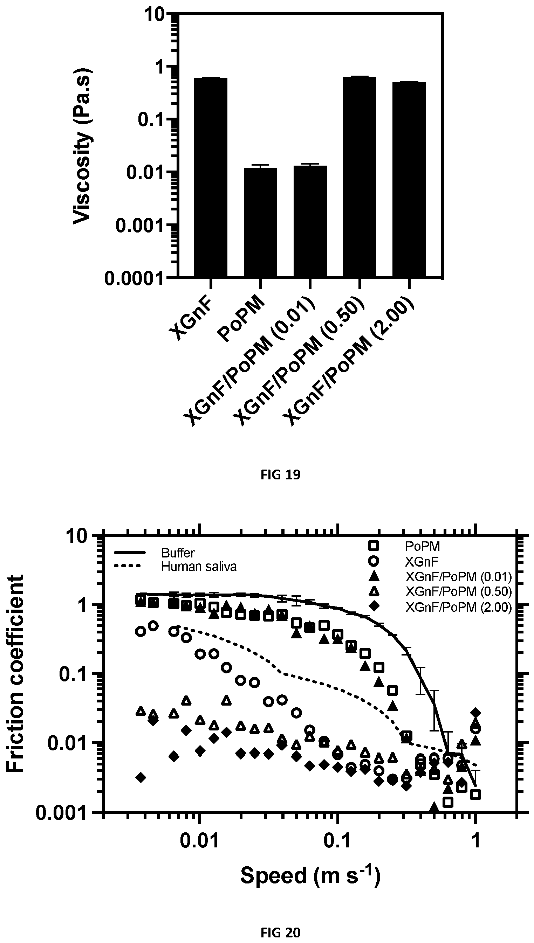

Figure 19 shows the shear viscosity at the orally relevant shear rate of PoPM, XGnF, and exemplary formulations of the invention made following Example 5 (comprising XGnF and PoPM in a ratio of 0.01:1 to 2:1 w/w). (The weight ratio of the one or more polysaccharide-based nanofibrils to the proteinaceous microgel of the formulations of the present invention is from about 0.1:1 to about 10:1.)

Figure 20 shows the friction coefficients as a function of speed obtained for exemplary formulations of the present invention made following Example 5 (comprising XGnF and PoPM in a ratio of 0.01:1 to 2:1 w/w), and compares these values with those obtained for PoPM, XGnF, human saliva and buffer. (The weight ratio of the one or more polysaccharide-based nanofibrils to the proteinaceous microgel of the formulations of the present invention is from about 0.1:1 to about 10:1.)

## DETAILED DESCRIPTION

The abbreviations used herein have their conventional meaning within the chemical and biological arts.

The invention is defined by the claims. Any subject-matter falling outside the scope of the claims is provided for informational purposes only. Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith, or not falling within the scope of the claims. All of the features disclosed in this specification (including any accompanying claims, abstract and drawings), and/or all of the steps of any method or process so disclosed, may be combined in any combination, except combinations where at least some of such features and/or steps are mutually exclusive, or which do not fall within the scope of the claims. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed, as long as these features, steps, and combinations thereof are according to the claims.

For the avoidance of doubt, it is hereby stated that the information disclosed earlier in this specification under the heading "Background" is useful to the understanding of the invention.

### Definitions

The term 'nanofibril' includes a tubular-shaped structure of any polymer with an equivalent diameter of approximately 1 to 100 nm.

The term 'colloidosome' includes a core-shell system having a colloidal core and a shell composed of colloidal particles or fibrils.

### Formulations

The proteinaceous microgel is positively charged and the one or more polysaccharide-based nanofibrils are negatively charged.

The one or more polysaccharide-based nanofibrils are associated with an outer surface of the proteinaceous microgel by an electrostatic interaction. The association of the polysaccharide-based nanofibrils with an outer surface of the proteinaceous microgel may be regarded as a form of 'coating' of the polysaccharide-based nanofibrils onto the outer surface of the proteinaceous microgel. The association (or coating) of the polysaccharide-based nanofibrils with an outer surface of the proteinaceous microgel results in an arrangement whereby the microgel is surrounded by a permeable mesh of polysaccharide-based nanofibrils of different local concentrations on the outer surface of the proteinaceous microgel. In an embodiment, the association between the polysaccharide-based nanofibrils and the outer surface of the proteinaceous microgel is a direct association, *i.e.,* the polysaccharide-based nanofibrils and the outer surface of the proteinaceous microgel are associated with one another in the absence of an intermediate component. In an embodiment, the formulation of the present invention consists of only two oppositely charged components *(i.e.,* the proteinaceous microgel and the one or more polysaccharide-based nanofibrils). As explained above, the two components of the formulation of the invention interact with each other *via* direct, electrostatic interactions, thus allowing the microgel particle to be coated with oppositely-charged polysaccharide-based nanofibrils.

In embodiments, the one or more polysaccharide-based nanofibrils associated with the outer surface of the proteinaceous microgel result in an outer surface that has an overall negative charge.

In embodiments, the proteinaceous microgel is selected from the group consisting of: lactoferrin, lysozyme, gelatin, such as gelatin Type B, milk protein, whey protein, caseinate, egg protein, albumin, such as bovine serum albumin, gluten, pea protein, rice protein, legumin, corn protein and peanut protein. In a preferred embodiment, the microgel is lactoferrin. In a preferred embodiment, the microgel is potato protein.

The proteinaceous microgel is charged at a pH of from about 3.0 to about 7.0. In such embodiments, the proteinaceous microgel may be selected from the group consisting of: lactoferrin, lysozyme, and gelatin Type B. Preferably, the microgel is charged at a pH of about 7.0.

In embodiments, the proteinaceous microgel is charged at a pH of from about 3.0 to about 4.0. In such embodiments, the protein may be selected from the group consisting of: gelatin, milk protein, whey protein, casein, caseinate, egg protein, albumin, such as bovine serum albumin, gluten, pea protein, potato protein, rice protein, legumin,

7

corn protein and peanut protein.

In embodiments, the proteinaceous microgel is charged at a pH of from about 3.0 to about 4.0. In such embodiments, the protein may be selected from the group consisting of: gelatin, milk protein, whey protein, caseinate, egg protein, albumin, such as bovine serum albumin, gluten, pea protein, rice protein, legumin, corn protein, and peanut protein.

In embodiments, the proteinaceous microgel has a diameter of about 100 nm to about 400 nm.

In embodiments, the proteinaceous microgel has a diameter of about 100 nm to about 300 nm.

In embodiments, the proteinaceous microgel has a diameter of about 100 nm to about 200 nm.

In a preferred embodiment, the one or more polysaccharide-based nanofibrils are κ-carrageenan nanofibrils. In a preferred embodiment, the one or more polysaccharide-based nanofibrils are made by addition of agar. In a preferred embodiment, the one or more polysaccharide-based nanofibrils are made by addition of xanthan gum.

According to the invention, the one or more polysaccharide-based nanofibrils have an equivalent diameter of from about 1 nm to about 100 nm. In embodiments, the one or more polysaccharide-based nanofibrils are no more than 50 nm in equivalent diameter.

In embodiments, the one or more polysaccharide-based nanofibrils have an equivalent diameter of from about 1 nm to about 50 nm. In embodiments, the one or more polysaccharide-based nanofibrils have an equivalent diameter of from about 5 nm to about 50 nm. In embodiments, the one or more polysaccharide-based nanofibrils have an equivalent diameter of from about 10 nm to about 50 nm.

In embodiments, the one or more polysaccharide-based nanofibrils have an equivalent diameter of from about 1 nm to about 40 nm. In embodiments, the one or more polysaccharide-based nanofibrils have an equivalent diameter of from about 1 nm to about 30 nm. In embodiments, the one or more polysaccharide-based nanofibrils have an equivalent diameter of from about 1 nm to about 20 nm.

In embodiments, the one or more polysaccharide-based nanofibrils have an equivalent diameter of from about 5 nm to about 40 nm. In embodiments, the one or more polysaccharide-based nanofibrils have an equivalent diameter of from about 5 nm to about 30 nm. In embodiments, the one or more polysaccharide-based nanofibrils have an equivalent diameter of from about 5 nm to about 20 nm.

In embodiments, the one or more polysaccharide-based nanofibrils have an equivalent diameter of from about 10 nm to about 40 nm. In embodiments, the one or more polysaccharide-based nanofibrils have an equivalent diameter of from about 10 nm to about 30 nm. In embodiments, the one or more polysaccharide-based nanofibrils have an equivalent diameter of from about 10 nm to about 20 nm.

Preferably, the one or more polysaccharide-based nanofibrils have an equivalent diameter of from about 5 nm to about 20 nm.

In embodiments, the one or more polysaccharide-based nanofibrils have a length of from about 75 nm to about 500 nm. In embodiments, the one or more polysaccharide-based nanofibrils have a length of from about 100 nm to about 500 nm.

In embodiments, the one or more polysaccharide-based nanofibrils have a length of from about 50 nm to about 400 nm. In embodiments, the one or more polysaccharide-based nanofibrils have a length of from about 75 nm to about 400 nm. In embodiments, the one or more polysaccharide-based nanofibrils have a length of from about 100 nm to about 400 nm.

In embodiments, the one or more polysaccharide-based nanofibrils have a length of from about 50 nm to about 300 nm. In embodiments, the one or more polysaccharide-based nanofibrils have a length of from about 75 nm to about 300 nm. In embodiments, the one or more polysaccharide-based nanofibrils have a length of from about 100 nm to about 300 nm.

In embodiments, the one or more polysaccharide-based nanofibrils have a length of from about 50 nm to about 475 nm. In embodiments, the one or more polysaccharide-based nanofibrils have a length of from about 50 nm to about 450 nm. In embodiments, the one or more polysaccharide-based nanofibrils have a length of from about 50 nm to about 425 nm. In embodiments, the one or more polysaccharide-based nanofibrils have a length of from about 50 nm to about 400 nm. In embodiments, the one or more polysaccharide-based nanofibrils have a length of from about 50 nm to about 375 nm. In embodiments, the one or more polysaccharide-based nanofibrils have a length of from about 50 nm to about 350 nm. In embodiments, the one or more polysaccharide-based nanofibrils have a length of from about 50 nm to about 325 nm. In embodiments, the one or more polysaccharide-based nanofibrils have a length of from about 50 nm to about 300 nm.

In embodiments, the one or more polysaccharide-based nanofibrils have a length of from about 75 nm to about 475 nm. In embodiments, the one or more polysaccharide-based nanofibrils have a length of from about 75 nm to about 450 nm. In embodiments, the one or more polysaccharide-based nanofibrils have a length of from about 75 nm to about 425 nm. In embodiments, the one or more polysaccharide-based nanofibrils have a length of from about 75 nm to about 400 nm. In embodiments, the one or more polysaccharide-based nanofibrils have a length of from about 75 nm to about 375 nm. In embodiments, the one or more polysaccharide-based nanofibrils have a length of from about 75 nm to about 350 nm. In embodiments, the one or more polysaccharide-based nanofibrils have a length of from about 75 nm to about 325 nm. In embodiments, the one or more polysaccharide-based nanofibrils have a length of from about 75 nm to about 300 nm.

In embodiments, the one or more polysaccharide-based nanofibrils have a length of from about 100 nm to about 475 nm. In embodiments, the one or more polysaccharide-based nanofibrils have a length of from about 100 nm to about 450 nm. In embodiments, the one or more polysaccharide-based nanofibrils have a length of from about 100 nm to about 425 nm. In embodiments, the one or more polysaccharide-based nanofibrils have a length of from about 100 nm to about 400 nm. In embodiments, the one or more polysaccharide-based nanofibrils have a length of from about 100 nm to about 375 nm. In embodiments, the one or more polysaccharide-based nanofibrils have a length of from about 100 nm to about 350 nm. In embodiments, the one or more polysaccharide-based nanofibrils have a length of from about 100 nm to about 325 nm. In embodiments, the one or more polysaccharide-based nanofibrils have a length of from about 100 nm to about 300 nm.

In a preferred embodiment, the one or more polysaccharide-based nanofibrils have a length of from about 100 nm to about 300 nm.

The % outer surface coverage is from about 50% to about 99% (*e.g.*, at least about 51%, at least about 52%, at least about 53% or at least about 54%). In embodiments, the % outer surface coverage is at least about 55% (*e.g.*, at least about 56%, at least about 57%, at least about 58% or at least about 59%). In embodiments, the % outer surface coverage is at least about 60% (*e.g.*, at least about 61%, at least about 62%, at least about 63% or at least about 64%). In embodiments, the % outer surface coverage is at least about 65% (*e.g.*, at least about 66%, at least about 67%, at least about 68% or at least about 69%). In embodiments, the % outer surface coverage is at least about 70% (*e.g.*, at least about 71%, at least about 72%, at least about 73% or at least about 74%). In embodiments, the % outer surface coverage is at least about 75% (*e.g.*, at least about 76%, at least about 77%, at least about 78% or at least about 79%). In embodiments, the % outer surface coverage is at least about 80% (*e.g.*, at least about 81%, at least about 82%, at least about 83% or at least about 84%). In embodiments, the % outer surface coverage is at least about 85% (*e.g.*, at least about 86%, at least about 87%, at least about 88% or at least about 89%). In embodiments, the % outer surface coverage is at least about 90%.

In embodiments the % outer surface coverage is from about 50% to about 95%. In embodiments, the % outer surface coverage is from about 50% to about 90%. In embodiments, the % outer surface coverage is from about 50% to about 85%. In embodiments, the % outer surface coverage is from about 50% to about 80%. In embodiments, the % outer surface coverage is from about 50% to about 75%. In embodiments, the % outer surface coverage is from about 50% to about 70%. In embodiments, the % outer surface coverage is from about 50% to about 60%.

In embodiments, the % outer surface coverage is from about 55% to about 99%. In embodiments, the % outer surface coverage is from about 55% to about 95%. In embodiments, the % outer surface coverage is from about 55% to about 90%. In embodiments, the % outer surface coverage is from about 55% to about 85%. In embodiments, the % outer surface coverage is from about 55% to about 80%. In embodiments, the % outer surface coverage is from about 55% to about 75%. In embodiments, the % outer surface coverage is from about 55% to about 70%. In embodiments, the % outer surface coverage is from about 55% to about 60%.

In embodiments, the % outer surface coverage is from about 60% to about 99%. In embodiments, the % outer surface coverage is from about 60% to about 95%. In embodiments, the % outer surface coverage is from about 60% to about 90%. In embodiments, the % outer surface coverage is from about 60% to about 85%. In embodiments, the % outer surface coverage is from about 60% to about 80%. In embodiments, the % outer surface coverage is from about 60% to about 75%. In embodiments, the % outer surface coverage is from about 60% to about 70%.

In embodiments, the % outer surface coverage is from about 65% to about 99%. In embodiments, the % outer surface coverage is from about 65% to about 95%. In embodiments, the % outer surface coverage is from about 65% to about 90%. In embodiments, the % outer surface coverage is from about 65% to about 85%. In embodiments, the % outer surface coverage is from about 65% to about 80%. In embodiments, the % outer surface coverage is from about 65% to about 75%. In embodiments, the % outer surface coverage is from about 65% to about 70%.

In embodiments, the % outer surface coverage is from about 70% to about 99%. In embodiments, the % outer surface coverage is from about 70% to about 95%. In embodiments, the % outer surface coverage is from about 70% to about 90%. In embodiments, the % outer surface coverage is from about 70% to about 85%. In embodiments, the % outer surface coverage is from about 70% to about 80%. In embodiments, the % outer surface coverage is from about 70% to about 75%.

In embodiments, the % outer surface coverage is from about 75% to about 99%. In embodiments, the % outer surface coverage is from about 75% to about 95%. In embodiments, the % outer surface coverage is from about 75% to about 90%. In embodiments, the % outer surface coverage is from about 75% to about 85%. In embodiments, the % outer surface coverage is from about 75% to about 80%.

In embodiments, the % outer surface coverage is from about 80% to about 99%. In embodiments, the % outer surface coverage is from about 80% to about 95%. In embodiments, the % outer surface coverage is from about 80% to about 90%. In embodiments, the % outer surface coverage is from about 80% to about 85%.

In embodiments, the % outer surface coverage is from about 85% to about 99%. In embodiments, the % outer surface coverage is from about 85% to about 95%. In embodiments, the % outer surface coverage is from about 85% to about 90%.

In embodiments, the % outer surface coverage is from about 90% to about 99%.

In embodiments, the formulation is a colloidosome. In embodiments, the colloidosome is no more than 1000 nm in diameter. In embodiments, the colloidosome has a diameter of from about 50 nm to about 1000 nm. In embodiments, the colloidosome has a diameter of from about 60 nm to about 1000 nm. In embodiments, the colloidosome has a diameter of about 70 nm to about 1000 nm. In embodiments, the colloidosome has a diameter of about 80 nm to about 1000 nm. In embodiments, the colloidosome has a diameter of about 90 nm to about 1000 nm. In embodiments, the colloidosome has a diameter of about 100 nm to about 1000 nm.

In embodiments, the colloidosome has a diameter of from about 50 nm to about 900 nm. In embodiments, the colloidosome has a diameter of from about 60 nm to about 900 nm. In embodiments, the colloidosome has a diameter of about 70 nm to about 900 nm. In embodiments, the colloidosome has a diameter of about 80 nm to about 900 nm. In embodiments, the colloidosome has a diameter of about 90 nm to about 900 nm. In embodiments, the colloidosome has a diameter of about 100 nm to about 900 nm.

In embodiments, the colloidosome has a diameter of from about 50 nm to about 800 nm. In embodiments, the colloidosome has a diameter of from about 60 nm to about 800 nm. In embodiments, the colloidosome has a diameter of about 70 nm to about 800 nm. In embodiments, the colloidosome has a diameter of about 80 nm to about 800 nm. In embodiments, the colloidosome has a diameter of about 90 nm to about 800 nm. In embodiments, the colloidosome has a diameter of about 100 nm to about 800 nm.

In embodiments, the colloidosome has a diameter of from about 50 nm to about 700 nm. In embodiments, the colloidosome has a diameter of from about 60 nm to about 700 nm. In embodiments, the colloidosome has a diameter of about 70 nm to about 700 nm. In embodiments, the colloidosome has a diameter of about 80 nm to about 700 nm. In embodiments, the colloidosome has a diameter of about 90 nm to about 700 nm. In embodiments, the colloidosome has a diameter of about 100 nm to about 700 nm.

In embodiments, the colloidosome has a diameter of from about 50 nm to about 600 nm. In embodiments, the colloidosome has a diameter of from about 60 nm to about 600 nm. In embodiments, the colloidosome has a diameter of about 70 nm to about 600 nm. In embodiments, the colloidosome has a diameter of about 80 nm to about 600 nm. In embodiments, the colloidosome has a diameter of about 90 nm to about 600 nm. In embodiments, the colloidosome has a diameter of about 100 nm to about 600 nm.

In embodiments, the formulation is a colloidosome. In embodiments, the colloidosome is no more than 500 nm in diameter. In embodiments, the colloidosome has a diameter of from about 50 nm to about 500 nm. In embodiments, the colloidosome has a diameter of from about 60 nm to about 500 nm. In embodiments, the colloidosome has a diameter of about 70 nm to about 500 nm. In embodiments, the colloidosome has a diameter of about 80 nm to about 500 nm. In embodiments, the colloidosome has a diameter of about 90 nm to about 500 nm. In embodiments, the colloidosome has a diameter of about 100 nm to about 500 nm.

In embodiments, the colloidosome is no more than 400 nm in diameter. In embodiments, the colloidosome has a diameter of from about 50 nm to about 400 nm. In embodiments, the colloidosome has a diameter of from about 60 nm to about 400 nm. In embodiments, the colloidosome has a diameter of about 70 nm to about 400 nm. In embodiments, the colloidosome has a diameter of about 80 nm to about 400 nm. In embodiments, the colloidosome has a diameter of about 90 nm to about 400 nm. In embodiments, the colloidosome has a diameter of about 100 nm to about 400 nm.

In embodiments, the colloidosome is no more than 300 nm in diameter. In embodiments, the colloidosome has a diameter of from about 50 nm to about 300 nm. In embodiments, the colloidosome has a diameter of from about 60 nm to about 300 nm. In embodiments, the colloidosome has a diameter of about 70 nm to about 300 nm. In embodiments, the colloidosome has a diameter of about 80 nm to about 300 nm. In embodiments, the colloidosome has a diameter of about 90 nm to about 300 nm. In embodiments, the colloidosome has a diameter of about 100 nm to about 300 nm.

In embodiments, the colloidosome has a diameter of no more than 200 nm. In embodiments, the colloidosome has a diameter of from about 50 nm to about 200 nm. In embodiments, the colloidosome has a diameter of from about 60 nm to about 200 nm. In embodiments, the colloidosome has a diameter of about 70 nm to about 200 nm. In a preferred embodiment, the colloidosome has a diameter of about 80 nm to about 200 nm. In embodiments, the colloidosome has a diameter of about 90 nm to about 200 nm. In embodiments, the colloidosome has a diameter of about 100 nm to about 200 nm.

In embodiments, the colloidosome has a diameter of about 80 nm. In embodiments, the colloidosome has a diameter of about 90 nm. In embodiments, the colloidosome has a diameter of about 100 nm. In embodiments, the colloidosome has a diameter of about 110 nm. In embodiments, the colloidosome has a diameter of about 120 nm. In embodiments, the colloidosome has a diameter of about 130 nm. In embodiments, the colloidosome has a diameter of about 140 nm. In embodiments, the colloidosome has a diameter of about 150 nm.

The % outer surface coverage of the microgel by the nanofibrils ( $c/c_{sat}$ ) is calculated by the following

equation: $\frac{3c}{c_{sat}} = -ln\left(\frac{\zeta_c - \zeta_{sat}}{\zeta_0 - \zeta_{sat}}\right)$ , wherein: $\zeta_{sat}$ is the $\zeta$-potential when the microgels are saturated with polysaccharide-based nanofibrils; $\zeta_0$ is he $\zeta$-potential of the proteinaceous microgel in absence of the polysaccharide-based nanofibrils; and $\zeta_c$ is the $\zeta$-potential of the formulation (*i.e.,* the colloidosome) at polysaccharide-based nanofibril concentration *c*. $c_{sat}$ is the minimum amount of the polysaccharide-based nanofibrils required to completely cover the surface of the proteinaceous microgel [Anges Teo, Sung Je Lee, Kelvin K. T. Goh, Food Structure, 2017, 14, 60-67; Anwesha Sarkar, Kelvin K.T. Goh, Harjinder Singh, Food Hydrocolloids, 2009, 23, 1270-1278; S. Pallandre, E. A. Decker and D. J. McClements, Journal of Food Science, 2007, 72, E518-E524; Demet Guzey and David Julian McClements, J. Agric. Food Chem., 2007, 55, 475-485].

The weight ratio of one or more polysaccharide-based nanofibrils to proteinaceous microgel is from about 0.1:1 to about 10:1. In embodiments, the weight ratio is from about 0.1:1 to about 5:1. In embodiments, the weight ratio is from about 0.1:1 to about 4:1. In embodiments, the weight ratio is from about 0.1:1 to about 3:1. In embodiments, the weight ratio is from about 0.1:1 to about 2:1. In embodiments, the weight ratio is from about 0.1:1 to about 1.5:1. In embodiments, the weight ratio is from about 0.1:1 to about 1:1.

In embodiments, the weight ratio of one or more polysaccharide-based nanofibrils to proteinaceous microgel is from about 0.2:1 to about 5:1. In embodiments, the weight ratio is from about 0.3:1 to about 5:1. In embodiments, the weight ratio is from about 0.4:1 to about 5:1. In embodiments, the weight ratio is from about 0.5:1 to about 5:1. In embodiments, the weight ratio is from about 0.6:1 to about 5:1. In embodiments, the weight ratio is from about 0.7:1 to about 5:1. In embodiments, the weight ratio is from about 0.8:1 to about 5:1. In embodiments, the weight ratio is from about 0.9:1 to about 5:1. In embodiments, the weight ratio is from about 1:1 to about 5:1. In embodiments, the weight ratio is from about 1.5:1 to about 5:1. In embodiments, the weight ratio is from about 2:1 to about 5:1. In embodiments, the weight ratio is from about 3:1 to about 5:1. In embodiments, the weight ratio is from about 4:1 to about 5:1.

In embodiments, the weight ratio of one or more polysaccharide-based nanofibrils to proteinaceous microgel is from about 0.2:1 to about 3:1. In embodiments, the weight ratio of one or more polysaccharide-based nanofibrils to proteinaceous microgel is from about 0.2:1 to about 2:1. In embodiments, the weight ratio is from about 0.3:1 to about 2:1. In embodiments, the weight ratio is from about 0.4:1 to about 2:1. In embodiments, the weight ratio is from about 0.5:1 to about 2:1. In embodiments, the weight ratio is from about 0.6:1 to about 2:1.

In embodiments, the weight ratio of one or more polysaccharide-based nanofibrils to proteinaceous microgel is from about 0.6:1 to about 1.5:1. In embodiments, the weight ratio is from about 0.6:1 to about 1:1.

In a preferred embodiment, the weight ratio is from about 0.2:1 to about 3:1.

In a preferred embodiment, the weight ratio is from about 0.6:1 to about 2:1.

In an embodiment, the formulation further comprises a pharmaceutically acceptable excipient.

In a preferred embodiment, the microgel is lactoferrin and the one or more polysaccharide-based nanofibrils are κ-carrageenan nanofibrils. In a preferred embodiment, the microgel is lactoferrin and the one or more polysaccharide-based nanofibrils are made by addition of agar. In a preferred embodiment, the microgel is potato protein and the one or more polysaccharide-based nanofibrils are κ-carrageenan nanofibrils. In a preferred embodiment, the microgel is potato protein and the one or more polysaccharide-based nanofibrils are made by addition of xanthan gum.

### *Method for preparing a formulation*

In embodiments, the buffer solution of step (a) may be selected from the group consisting of: 4-(2-hydroxyethyl)-1-piperazineethanesulfonic acid (HEPES), phosphate buffer, 2-(N-Morpholino)ethanesulfonic acid hydrate, 4-Morpholineethanesulfonic acid (MES hydrate), 2,2-Bis(hydroxymethyl)-2,2',2"-nitrilotriethanol (Bis-Tris), citric acid monohydrate and trisodium citrate dihydrate.

In embodiments, the buffer solution of step (a) may be selected from the group consisting of: 4-(2-hydroxyethyl)-1-piperazineethanesulfonic acid (HEPES), phosphate buffer, 2-(N-Morpholino)ethanesulfonic acid hydrate, 4-Morpholineethanesulfonic acid (MES hydrate), 2,2-Bis(hydroxymethyl)-2,2',2"-nitrilotriethanol (Bis-Tris) and citric acid monohydrate.

In embodiments, the buffer solution of step (a) has a concentration of from about 1 to 50 mM. In an embodiment, the buffer solution of step (a) has a concentration of about 20 mM. In a preferred embodiment, the buffer solution of step (a) has a concentration of about 10 mM.

The buffer solution of step (a) has a pH of from about 3.0 to about 7.0. In an embodiment, the buffer solution of step (a) has a pH of from about 3.0 to about 4.0. In a preferred embodiment, the buffer solution of step (a) has a pH of about 7.0. In a preferred embodiment, the buffer solution of step (a) has a pH of about 3.0.

The proteinaceous microgel is selected from the group consisting of: lactoferrin, lysozyme, gelatin, such as gelatin Type B, milk protein, whey protein, casein, caseinate, egg protein, albumin, such as bovine serum albumin, gluten, pea protein, rice protein, legumin, corn protein, peanut protein and potato protein. In embodiments, the proteinaceous microgel is selected from the group consisting of: lactoferrin, lysozyme, gelatin, such as gelatin Type B, milk protein, whey protein, caseinate, egg protein, albumin, such as bovine serum albumin, gluten pea protein, rice protein, legumin, corn protein and peanut protein. In a preferred embodiment, the proteinaceous microgel is lactoferrin. In a preferred embodiment, the proteinaceous microgel is potato protein.

In embodiments, the resulting solution of step (a) comprises the proteinaceous material in an amount of at least about 4 wt%. In embodiments, the resulting solution of step (a) comprises the proteinaceous material in an amount of at least about 6 wt%. In embodiments, the resulting solution of step (a) comprises the proteinaceous material in an amount of at least about 8 wt%. In embodiments, the resulting solution of step (a) comprises the proteinaceous material in an amount of no more than about 20 wt%.

In a preferred embodiment, the resulting solution of step (a) comprises the proteinaceous material in an amount of about 12 wt%. In a preferred embodiment, the resulting solution of step (a) comprises the proteinaceous material in an amount of about 9 wt%. In a preferred embodiment, the resulting solution of step (a) comprises the proteinaceous material in an amount of about 6 wt%.

In embodiments, dissolving the proteinaceous material in the buffer solution in step (a) comprises stirring the mixture until complete solubilisation occurs. In embodiments, dissolving the proteinaceous material in the buffer solution in step (a) involves stirring the mixture for at least about 5 minutes, for at least about 20 minutes, for at least about 30 minutes, for at least about 40 minutes, for at least about 50 minutes, for at least about 1 hour, for at least about 1.5 hours, for at least about 2 hours, or for at least about 2.5 hours.

In a preferred embodiment, dissolving the proteinaceous material in the buffer solution in step (a) involves stirring the mixture for about 2 hours.

In embodiments, heating the resulting solution in step (a) is performed for at least about 10 minutes, for at least about 20 minutes or for at least about 30 minutes.

In a preferred embodiment, heating the resulting solution in step (a) is performed for about 30 minutes.

In embodiments, heating the resulting solution in step (a) is performed at a temperature of at least about 65 ° C (*e.g.,* at least about 65 ° C, at least about 70 ° C, at least about 75 ° C, or at least about 80 ° C). In embodiments, heating the resulting solution in step (a) is performed at a temperature of at least about 70 ° C (*e.g.,* at least about 75 ° C, at least about 80 ° C, at least about 85 ° C, or at least about 90 ° C). In embodiments, heating the resulting solution in step (a) is performed at a temperature of at least about 65 ° C and no more than about 150 ° C (*e.g.*, at least about 65° C and no more than about 140 ° C, at least about 70° C and no more than about 130 ° C or at least about 80° C and no more than about 110 ° C. In embodiments, heating the resulting solution in step (a) is performed at a temperature of at least about 70 ° C and no more than about 150 ° C (*e.g.*, at least about 70° C and no more than about 140 ° C, at least about 80° C and no more than about 130 ° C or at least about 90° C and no more than about 110 ° C.

In a preferred embodiment, heating the resulting solution is performed at about 90 ° C. In a preferred embodiment, heating the resulting solution is performed at about 65 ° C.

In embodiments, the weight ratio of heat-set gel to buffer solution in step (b) is about 3:1 w/w.

In embodiments, the step of homogenising to form the proteinaceous microgel in step (b) is performed at a pressure of at least 300 bar.

In embodiments, step (b) further comprises the step of blending the mixture of heat-set gel and buffer solution to form macrogel particles before homogenising to form the proteinaceous microgel.

In embodiments, step (b) further comprises the step of degassing the mixture of heat-set gel and buffer solution before homogenising to form the proteinaceous microgel. In such embodiments, the mixture is degassed for at least about 3 minutes.

In embodiments, the solution of one or more polysaccharide-based nanofibrils of step (c) comprises at least about 0.05 wt% of the one or more polysaccharide-based nanofibrils. In embodiments, the solution of one or more polysaccharide-based nanofibrils of step (c) comprises no more than about 5 wt% of the one or more polysaccharide-based nanofibrils. In embodiments, the solution of one or more polysaccharide-based nanofibrils of step (c) comprises from about 0.05 wt% to about 3 wt % of the one or more polysaccharide-based nanofibrils.

In a preferred embodiment, the solution of one or more polysaccharide-based nanofibrils of step (c) comprises about 1.5 wt% of the one or more polysaccharide-based nanofibrils.

In embodiments, the buffer solution of step (c) may be selected from the group consisting of: 4-(2-hydroxyethyl)-1-piperazineethanesulfonic acid (HEPES), phosphate buffer, 2-(N-Morpholino)ethanesulfonic acid hydrate, 4-Morpholineethanesulfonic acid (MES hydrate), 2,2-Bis(hydroxymethyl)-2,2',2"-nitrilotriethanol (Bis-Tris), citric acid monohydrate and trisodium citrate dihydrate.

In embodiments, the solution of one or more polysaccharide-based nanofibrils of step (c) comprises a buffer solution. In such embodiments, the buffer solution may be selected from the group consisting of: 4-(2-hydroxyethyl)-1-piperazineethanesulfonic acid (HEPES), phosphate buffer, 2-(N-Morpholino)ethanesulfonic acid hydrate, 4-Morpholineethanesulfonic acid (MES hydrate), 2,2-Bis(hydroxymethyl)-2,2',2"-nitrilotriethanol (Bis-Tris) and citric acid monohydrate.

In such embodiments, the buffer solution of step (c) has a concentration of from about 1 mM to 50 mM. In an embodiment, the buffer solution of step (c) has a concentration of about 20 mM. In a preferred embodiment, the buffer solution of step (c) has a concentration of about 10 mM.

In embodiments, the weight ratio of the proteinaceous microgel to one or more polysaccharide-based nanofibrils used in step (c) is selected in accordance with paragraphs [0072] to [0077].

The solution of one or more polysaccharide-based nanofibrils of step (c) is formed by (i) heating a mixture of one or more polysaccharide-based materials and buffer solution while shearing the mixture to form the one or more polysaccharide-based nanofibrils, and (ii) cooling the resulting aqueous dispersion comprising the nanofibrils. In such embodiments, heating the mixture in step (i) may be performed at a temperature of at least about 50 °C (e.g., at least about 60 °C, at least about 70 °C, at least about 80 °C, at least about 90 °C). In such embodiments, cooling the resulting aqueous dispersion comprising the one or more polysaccharide-based nanofibrils in step (ii) may be performed at around 37 °C.

### Uses

In an aspect of the invention, there is provided a use of the formulation of the invention as a lubricant food additive, i.e. for fat replacement purposes. In embodiments, the use includes applying a formulation of the invention to food in a concentration of from about 5 to about 90%.

In embodiments, the use involves the addition of the lubricant food additive to a beverage or solid food selected from the group consisting of: chewing gum, candy, chocolate and frozen food products.

## EXAMPLES

### Materials and Methods

Lactoferrin was purchased from Ingredia, France; $\kappa$-carrageenan was purchased from Sigma-Aldrich, UK; agar was purchased from Scientific Laboratory Supplies, UK; potato protein was purchased from Sosa Ingredients, Spain; xanthan gum was purchased from Sigma-Aldrich, UK. Biopolymers, lactoferrin, potato protein isolate, $\kappa$-carrageenan, agar and xanthan gum were made in pH 7.0 buffer consisting of 10 mM 4-(2-hydroxyethyl)-1-piperazineethanesulfonic acid (HEPES), the pH was adjusted to salivary pH (pH 7.0) by adding NaOH, or in pH 3.0 buffer consisting of 10 mM citric acid monohydrate and 10 mM trisodium citrate dihydrate mixed in adequate proportions so as to reach an acidic pH (pH 3.0). Milli-Q water purified by treatment with a Milli-Q apparatus (Millipore Corp., Bedford, MA, USA) was used to prepare the buffer.

### Characterisation

#### $\zeta$-potential measurement

The $\zeta$-potential of samples at pH 7.0 or pH 3.0 were measured by Zetasizer (Nano ZS series, Malvern Instruments Ltd., UK). The samples were added into folded electrophoretic cells (DTS1070, Malvern Instruments Ltd., UK) at 25 °C and diluted 100 times before the measurement.

#### Transmission electron microscopy

Transmission electron microscopy of samples was performed using a transmission electron microscope (Tecnai G2 Spirit-T12, ThermoFisher, USA). Voltage of the electron gun was fixed at 120 kV and images were captured using a Gantan CCD camera. In order to increase the electron contrast, the samples were negatively stained. For this purpose, 5.0 $\mu$L of the samples were deposited on a carbon coated TEM grid. Before depositing the samples, the grid was electrostatically cleaned using a Pelco easyGlow discharge cleaning system (Ted Pella, Inc., USA). After deposition, the samples were left to rest for 60 s and excess of the sample at the edge of the grid was removed using a filter paper. Samples were stained by adding 5.0 $\mu$L of 1.0% uranyl acetate for 10 s and the excess of uranyl acetate was removed. The treatment with uranyl acetate was repeated twice and the samples were then air-dried before imaging.

#### Rheology

A modular compact controlled-stress rheometer (MCR-302, Anton Paar, Austria) was used to measure the apparent viscosity of all samples, equipped with a cone-plate geometry (CP50-1, diameter 50 mm, angle 1°). The gap size corresponding to this geometry was 0.208 mm. Viscosity measurements were performed in a range of shear rates from 0.1 $s^{-1}$ to 100 $s^{-1}$ at a fixed temperature of 37 °C. The data points were set to be 6 points/decade, and the duration was set by the device to ensure reaching steady state for each point. In addition, the rheology of real human saliva (MEEC 16-046, ethics approved by Faculty Ethics Committee, University of Leeds) and honey was used as controls.

Tribology

A Mini Traction Machine (MTM2, PCS Instruments, UK) was used to measure the lubrication properties of all samples, with hydrophobic polydimethylsiloxane (PDMS) ball (Ø 19 mm)-on-disk (Ø 46 mm) configuration mimicking the hydrophobic tongue-palate of dry mouth. The surface roughness $R_a$ of PDMS (Sylgard 184, Dow Corning, USA) was 50 nm. The temperature was set at 37 °C and the load was fixed at 2.0 N for all experiments. In addition, the tribology of real human saliva (MEEC 16-046, ethics approved by Faculty Ethics Committee, University of Leeds) was used as controls.

With this invention, the inventors demonstrate formulations comprising proteinaceous microgels partially coated with polysaccharide-based nanofibrils. These formulations achieve better lubrication performance than commercial lubricants and human saliva, and provide lowering of friction coefficients without the need of high viscosity.

Example 1: Manufacture of lactoferrin microgels coated by κ-carrageenan nanofibrils

KCnF/LFM - 140 nm

κ-carrageenan nanofibrils (KCnF) were prepared by dissolving κ-carrageenan powder in 10 mM HEPES buffer (mentioned above) by heating at 95 ° C while being sheared for 40 minutes under constant stirring for a complete solubilisation and formation of nanofibrils. This aqueous dispersion containing KCnF was then cooled to around 37 ° C.

Lactoferrin solution (12 wt%) was prepared by adding lactoferrin powder in 10 mM HEPES buffer at pH 7.0 and stirring for 2 hours to ensure complete solubilisation. The solution was heated at 90 ° C for 30 minutes to form heat-set gel, which was mixed with 10 mM HEPES buffer (3:1 w/w) at pH 7.0 and broken into macrogel particles using a hand blender (HB724, Kenwood, UK) for 5 minutes. Then the resulting lactoferrin macrogel particles + buffer mixture was transferred to a conditioning mixer (ARE-250, THINKY Corporation, Japan) for degassing for 3 minutes. The degassed macrogel particle + buffer mixture was then homogenized by passing twice through Leeds Jet Homogenizer operating at a pressure of 300 ± 20 bars to form lactoferrin microgel (LFM) particles.

The formulation was prepared by adding LFM to KCnF under gentle stirring at different weight ratios ranging from 0.01:1 to 3:1 w/w (KCnF/LFM).

The different weight ratios are illustrated in the following Table:

| KCnF in formulation (wt%) | LFM in formulation (wt%) | Ratio of KCnF/LFM (wt/wt) |
|---|---|---|
| 0.02 | 2.00 | 0.01* |
| 0.07 | 2.00 | 0.03* |
| 0.14 | 2.00 | 0.07* |
| 0.40 | 2.00 | 0.20 |
| 0.80 | 2.00 | 0.40 |
| 1.16 | 2.00 | 0.60 |
| 1.16 | 1.16 | 1.00 |
| 1.16 | 0.58 | 2.00 |
| 1.16 | 0.39 | 3.00 |
| *Outside the scope of the claimed invention. | | |

Example 2: Manufacture of lactoferrin microgels coated by κ-carrageenan nanofibrils

KCnF/LFM - 90 nm

κ-carrageenan nanofibrils (KCnF) were prepared by dissolving κ-carrageenan powder in 10 mM HEPES buffer (mentioned above) at pH 7.0 by heating at 95 ° C while being sheared for 40 minutes under constant stirring for a complete solubilisation and formation of nanofibrils. This aqueous dispersion containing KCnF was then cooled to around 37 ° C.

Lactoferrin solution (9 wt%) was prepared by adding lactoferrin powder in 10 mM HEPES buffer at pH 7.0 and stirring for 2 hours to ensure complete solubilisation. Then the solution was heated at 90 ° C for 30 minutes to form lactoferrin microgel (LFM) particles.

The formulation was prepared by adding LFM to KCnF under gentle stirring at different weight ratios ranging from 0.01:1 to 2:1 w/w (KCnF/LFM). The different weight ratios are illustrated in the following Table:

| KCnF in formulation (wt%) | LFM in formulation (wt%) | Ratio of KCnF/LFM (wt/wt) |
|---|---|---|
| 0.02 | 2.00 | 0.01* |
| 1.16 | 2.00 | 0.60 |
| 1.16 | 1.16 | 1.00 |
| 1.16 | 0.58 | 2.00 |
| *Outside the scope of the claimed invention. | | |

Example 3: Manufacture of lactoferrin microgels coated by agar nanofibrils

AnF/LFM - 90 nm

Agar nanofibrils (AnF) were prepared by dissolving agar powder in 10 mM HEPES buffer (mentioned above) at pH 7.0 by heating at 95 ° C while being sheared for 40 minutes under constant stirring for a complete solubilisation and formation of nanofibrils. This aqueous dispersion containing AnF was then cooled to around 37 ° C.

Lactoferrin solution (9 wt%) was prepared by adding lactoferrin powder in 10 mM HEPES buffer at pH 7.0 and stirring for 2 hours to ensure complete solubilisation. Then the solution was heated at 90 °C for 30 minutes to form lactoferrin microgel (LFM) particles.

The formulation was prepared by adding LFM to AnF under gentle stirring at different weight ratios ranging from 0.01:1 to 1:1 w/w (AnF/LFM). The different weight ratios are illustrated in the following Table:

| AnF in formulation (wt%) | LFM in formulation (wt%) | Ratio of AnF/LFM (wt/wt) |
|---|---|---|
| 0.02 | 2.00 | 0.01* |
| 1.16 | 1.16 | 1.00 |
| *Outside the scope of the claimed invention. | | |

Example 4: Manufacture of potato protein microgels coated by κ-carrageenan nanofibrils

KCnF/PoPM - 100 nm

κ-carrageenan nanofibrils (KCnF) were prepared by dissolving κ-carrageenan powder in 10 mM citrate buffer (mentioned above) at pH 3.0 by heating at 95 ° C while being sheared for 40 minutes under constant stirring for a complete solubilisation and formation of nanofibrils. This aqueous dispersion containing KCnF was then cooled to around 37 ° C.

Potato protein isolate solution (6 wt%) was prepared by adding potato protein isolate powder in 10 mM citrate buffer at pH 3.0 and stirring for 2 hours to ensure complete solubilisation. Then the pH of the solution was adjusted to 3.0 by adding HCl and finally the solution was heated at 65 ° C for 30 minutes to form potato protein microgel (PoPM) particles.

The formulation was prepared by adding PoPM to KCnF under gentle stirring at different weight ratios ranging from 0.01:1 to 2:1 w/w (KCnF/PoPM). The different weight ratios are illustrated in the following Table:

| KCnF in formulation (wt%) | PoPM in formulation (wt%) | Ratio of KCnF/PoPM (wt/wt) |
|---|---|---|
| 0.02 | 2.00 | 0.01* |
| 1.00 | 2.00 | 0.50 |
| 1.16 | 0.58 | 2.00 |
| *Outside the scope of the claimed invention. | | |

Example 5: Manufacture of potato protein microgels coated by xanthan gum nanofibrils

XGnF/PoPM - 100 nm

Xanthan gum nanofibrils (XGnF) were prepared by dissolving xanthan gum powder in 10 mM citrate buffer at pH 3.0 (mentioned above) at room temperature while being sheared for 24 hours under constant stirring for a complete solubilisation, hydration and formation of nanofibrils.

Potato protein isolate solution (6 wt%) was prepared by adding potato protein isolate powder in 10 mM citrate buffer at pH 3.0 and stirring for 2 hours to ensure complete solubilisation. Then the pH of the solution was adjusted to 3.0 by adding HCl and finally the solution was heated at 65 ° C for 30 minutes to form potato protein microgel (PoPM) particles.

The formulation was prepared by adding PoPM to XGnF under gentle stirring at different weight ratios ranging from 0.01:1 to 2:1 w/w (KCnF/PoPM). The different weight ratios are illustrated in the following Table:

| XGnF in formulation (wt%) | PoPM in formulation (wt%) | Ratio of XGnF/PoPM (wt/wt) |
|---|---|---|
| 0.02 | 2.00 | 0.01* |
| 1.00 | 2.00 | 0.50 |
| 1.16 | 0.58 | 2.00 |
| *Outside the scope of the claimed invention. | | |

Example 6: Analysis of lactoferrin microgels coated by κ-carrageenan nanofibrils manufactured in Example 1

A ratio of KCnF : LFM of 0.6 : 1 was selected for the lactoferrin microgels coated by κ-carrageenan nanofibrils used in the following analysis.

The transmission electron micrographs in Figure 1a show LFM particles as circular dark areas with diameters of less than 300 nm. In Figure 1b, KCnF show an average diameter and length of 10-20 nm and 100-300 nm, respectively. Figures 1c and 1d show LFM particles covered by KCnF. KCnF are also seen in the continuous phase connecting different colloidosome subunits.

Figure 2a shows the schematic representation of the colloidosome composed by LFM particles that are coated by KCnF. Under uniaxial tensile deformation, the colloidosome forms a macroscopic filament spanning the surfaces applying the deformation (Figures 2b and 2c). This kind of structure under tensile testing is commonly shown by polymer melts and solutions, and is an important feature of human saliva.

The $\zeta$-potential decreases upon increasing the concentration of KCnF (negatively charged) relatively to the concentration of LFM (positively charged) (Figure 3). Raising the ratio, $i.e.,$ increasing the concentration of KCnF, allows the transition of the $\zeta$-potential from positive to negative. In other words, upon increasing KCnF/LFM ratio, LFM particles become gradually negatively charged due to the gradual coverage by KCnF as shown in the table below:

$\zeta$-potential of LFM, KCnF and colloidosomes

| | LFM | KCnF | KCnF/LFM (0.6:1 w/w) |
|---|---|---|---|
| $\zeta$-potential (mV) | +22.0 | -46.3 | -41.5 |

To evaluate the fluidity of the colloidosomes under relevant oral conditions, the apparent viscosity of our samples at various ratios at an orally relevant shear rate was compared (Figure 4). For comparative purposes, the fluidity of two of our samples was compared with real human saliva, honey and various commercially available formulations at an orally relevant shear rate (Figure 5). In comparison to high viscosity fluid food products, such as honey and certain commercial formulations, the viscosity of the new colloidosome was one order of magnitude lower, indicating the good fluidity of the particle mixture.

Figure 6 shows the lubrication performance of the different colloidosomes under orally relevant conditions, represented by the friction coefficient as a function of speed. For comparative purposes, the lubrication performance of two of our samples was compared with real human saliva, buffer and various commercially available formulations under orally relevant conditions (Figure 7).

In comparison to buffer, both LFM and KCnF decrease the friction coefficient at orally relevant speeds ranging from 0.004 to 0.1 m/s by at least two folds (Figure 6). However, in comparison to real human saliva, friction coefficients obtained for both are twice as high in the boundary regime. A good salivary substitute is expected to surpass the tribological performance of real human saliva in both the boundary and fluid film regimes. Therefore, the reduction obtained by the components separately is not enough.

Additionally, the formulations of the invention demonstrate improved friction coefficients across all speeds tested unlike the commercially available lubricants (Figure 7). At lower speeds, Biotène® Oral Balance Moisturising Gel has comparable friction coefficients to that exhibited by the present invention, but at higher speeds, friction coefficients are far worse than real human saliva. At higher speeds, BioXtra Dry Mouth Gel Mouthspray, Boots Expert Dental Mouthspray, A.S Saliva Orthana Oral Spray and some of Glandosane sprays have comparable friction coefficients to that exhibited by the present invention, but at lower speeds, friction coefficients are far worse.

Summary:

The formulations of the present invention, *i.e.,* KCnF-coated LFM, is capable to provide a reduction in friction coefficients in comparison to real human saliva, throughout the entire orally relevant speeds. However, on decreasing KCnF:LFM ratio, friction coefficients increase back higher than real human saliva, which is in agreement with the $\zeta$-potential measurements (Figure 3).

Example 7: Analysis of lactoferrin microgels coated by $\kappa$-carrageenan nanofibrils manufactured in Example 2

The $\zeta$-potential decreases upon increasing the concentration of KCnF (negatively charged) relatively to the concentration of LFM (positively charged) (Figure 8). Raising the ratio, *i.e.,* increasing the concentration of KCnF, allows the transition of the $\zeta$-potential from positive to negative. In other words, upon increasing KCnF/LFM ratio, LFM particles become gradually negatively charged due to the gradual coverage by KCnF.

To evaluate the fluidity of the colloidosomes under relevant oral conditions, the apparent viscosity of our samples at various ratios at an orally relevant shear rate was compared (Figure 9).

Figure 10 shows the lubrication performance of the different colloidosomes under orally relevant conditions, represented by the friction coefficient as a function of speeds. The lubrication properties of buffer and real human saliva are also shown for comparison purposes.

Figure 11 shows the lubrication performance of the different colloidosomes under orally relevant conditions, represented by the friction coefficient as a function of speeds, at 0 month, 1 month and 2 months storage. This demonstrates that the lubricants are stable.

Example 8: Analysis of lactoferrin microgels coated by agar nanofibrils manufactured in Example 3

The $\zeta$-potential decreases upon increasing the concentration of AnF (negatively charged) relatively to the concentration of LFM (positively charged) (Figure 12). Raising the ratio, *i.e.,* increasing the concentration of AnF, allows the transition of the $\zeta$-potential from positive to negative. In other words, upon increasing AnF/LFM ratio, LFM particles become gradually negatively charged due to the gradual coverage by AnF.

To evaluate the fluidity of the colloidosomes under relevant oral conditions, the apparent viscosity of our samples at various ratios at an orally relevant shear rate was compared (Figure 13).

Figure 14 shows the lubrication performance of the different colloidosomes under orally relevant conditions, represented by the friction coefficient as a function of speeds. The lubrication properties of buffer and real human saliva are also shown for comparison purposes.

Example 9: Analysis of potato protein microgels coated by $\kappa$-carrageenan nanofibrils manufactured in Example 4

The $\zeta$-potential decreases upon increasing the concentration of KCnF (negatively charged) relatively to the concentration of PoPM (positively charged) (Figure 15). Raising the ratio, *i.e.,* increasing the concentration of KCnF, allows the transition of the $\zeta$-potential from positive to negative. In other words, upon increasing KCnF/PoPM ratio, PoPM particles become gradually negatively charged due to the gradual coverage by KCnF.

To evaluate the fluidity of the colloidosomes under relevant oral conditions, the apparent viscosity of our samples at various ratios at an orally relevant shear rate was compared (Figure 16).

Figure 17 shows the lubrication performance of the colloidosomes under orally relevant conditions, represented by the friction coefficient as a function of speeds. The lubrication properties of buffer and real human saliva are also shown for comparison purposes.

Example 10: Analysis of potato protein microgels coated by xanthan gum nanofibrils manufactured in Example 5

The $\zeta$-potential decreases upon increasing the concentration of XGnF (negatively charged) relatively to the concentration of PoPM (positively. charged) (Figure 18). Raising the ratio, *i.e.,* increasing the concentration of XGnF, allows the transition of the ζ-potential from positive to negative. In other words, upon increasing XGnF/PoPM ratio, PoPM particles become gradually negatively charged due to the gradual coverage by XGnF.

To evaluate the fluidity of the colloidosomes under relevant oral conditions, the apparent viscosity of our samples at various ratios at an orally relevant shear rate was compared (Figure 19).

Figure 20 shows the lubrication performance of the colloidosomes under orally relevant conditions, represented by the friction coefficient as a function of speeds. The lubrication of buffer and real human saliva are also shown for comparison purposes.

Example 11: Surface coverage

The ζ-potential of the colloidosomes of different formulations was measured. From those measurements, the % outer surface coverage can be calculated by the following equation:

$$\frac{3c}{c_{sat}} = -ln\left(\frac{\zeta_c - \zeta_{sat}}{\zeta_0 - \zeta_{sat}}\right)$$

The following table illustrates the % outer surface coverage in colloidosomes of different formulations:

| Ratio of KCnF/LFM (w/w) | 0.01* | 0.03* | 0.04* | 0.06* | 0.07* | 0.09* | 0.11 | 0.6 | 1.0 | 3.0 |
|---|---|---|---|---|---|---|---|---|---|---|
| % outer surface coverage *(c/c<sub>sat</sub>)* | 0.3 | 0.5 | 0.7 | 1.2 | 37.8 | 42.4 | 53 | 84.1 | 86.1 | 99.0 |
| *Outside the scope of the claimed invention. | | | | | | | | | | |

**Claims**

1. A method for preparing a formulation, the method comprising:

(a) dissolving a proteinaceous material in a buffer solution and heating the resulting solution to form a proteinaceous microgel or a heat-set gel;
(b) when step (a) results in a heat-set gel, mixing the heat-set gel with the buffer solution and homogenising to form a proteinaceous microgel, wherein the proteinaceous microgel is selected from the group consisting of: lactoferrin, lysozyme, gelatin, such as gelatin Type B, milk protein, whey protein, casein, caseinate, egg protein, albumin, such as bovine serum albumin, gluten, pea protein, rice protein, legumin, corn protein, peanut protein and potato protein, wherein the proteinaceous microgel has a diameter of about 100 nm to about 500 nm;
(c) preparing a solution of one or more polysaccharide-based nanofibrils by: (i) heating a mixture of one or more polysaccharide materials and a buffer solution while shearing the mixture to form the one or more polysaccharide-based nanofibrils, (ii) cooling the resulting aqueous dispersion comprising the nanofibrils; and
(d) adding the proteinaceous microgel of step (a) or step (b) to the solution of one or more polysaccharide-based nanofibrils of step (c) to form the formulation, wherein the polysaccharide-based nanofibrils selected from the group consisting of: κ-carrageenan, ɩ-carrageenan, λ-carrageenan, agar, agarose, alginate, pectin, dextran sulphate, cellulose, xanthan gum, gellan gum and any negatively-charged polysaccharide; wherein the one or more polysaccharide-based nanofibrils have an equivalent diameter of from about 1 nm to about 100 nm and a length of from about 50 nm to about 500 nm,
wherein the proteinaceous microgel is positively charged having a pH of from about 3.0 to about 7.0, and the one or more the polysaccharide-based nanofibrils is negatively charged;
wherein the weight ratio of one or more polysaccharide-based nanofibrils to proteinaceous microgel is from about 0.1:1 to about 10:1;
wherein the resulting formulation has the one or more polysaccharide-based nanofibrils associated with an outer surface of the proteinaceous microgel; and
wherein the amount of microgel that is added to nanofibrils is selected such that the % outer surface coverage of the microgel by the nanofibrils is from about 50% to about 99%, wherein the % outer surface coverage of the microgel by the nanofibrils ( $^{c}/_{c_{sat}}$ ) is calculated by the following equation:

$$\frac{3c}{c_{sat}} = -ln\left(\frac{\zeta_c - \zeta_{sat}}{\zeta_0 - \zeta_{sat}}\right)$$

wherein:

$\zeta_{sat}$ is the $\zeta$-potential when the microgels are saturated with polysaccharide-based nanofibrils;
$\zeta_0$ is the $\zeta$-potential of the proteinaceous microgel in absence of the polysaccharide-based nanofibrils;
$\zeta_c$ is the $\zeta$-potential of the formulation at polysaccharide-based nanofibril concentration c; and

$c_{sat}$ is the minimum amount of the polysaccharide-based nanofibrils required to completely cover the surface of the proteinaceous microgel.

2. The method of claim 1, wherein the weight ratio of one or more polysaccharide-based nanofibrils to proteinaceous microgel is from about 0.2:1 to about 3:1.

3. The method of any preceding claim, wherein the proteinaceous microgel is a lactoferrin microgel.

4. The method of any preceding claim, wherein the one or more polysaccharide-based nanofibrils are κ-carrageenan nanofibrils.

5. The method of any preceding claim, wherein the resulting formulation is a colloidosome, optionally wherein the colloidosome is no more than 1000 nm in diameter.

6. The method of any preceding claim, wherein the proteinaceous microgel has a pH of from about 3.0 to about 4.0, or of about 7.0.

7. A formulation obtainable or obtained by the method of any preceding claim.

8. A use of a formulation as a lubricant food additive; or a formulation for use in the treatment of a disease or condition selected from: dry mouth, xerostomia, or dysphagia;

optionally wherein the dry mouth is caused by: salivary gland diseases and disorders, chronic inflammatory autoimmune diseases, Sjögren's syndrome, endocrine diseases, diabetes, neurologic diseases and disorders, psychogenic diseases, anxiety, nervousness, HIV/AIDS and polypharmacy;
wherein the formulation comprises:

(i) a proteinaceous microgel, selected from the group consisting of: lactoferrin, lysozyme, gelatin, such as gelatin Type B, milk protein, whey protein, casein, caseinate, egg protein, albumin, such as bovine serum albumin, gluten, pea protein, rice protein, legumin, corn protein, peanut protein and potato protein; wherein the proteinaceous microgel has a diameter of about 100 nm to about 500 nm; and
(ii) one or more polysaccharide-based nanofibrils, selected from the group consisting of: κ-carrageenan, ι-carrageenan, λ-carrageenan, agar, agarose, alginate, pectin, dextran sulphate, cellulose, xanthan gum, gellan gum and any negatively-charged polysaccharide; wherein the one or more polysaccharide-based nanofibrils have an equivalent diameter of from about 1 nm to about 100 nm and a length of from about 50 nm to about 500 nm;

wherein the proteinaceous microgel is positively charged having a pH of from about 3.0 to about 7.0, and the one or more polysaccharide-based nanofibrils is negatively charged;
wherein the one or more polysaccharide-based nanofibrils are associated with an outer surface of the oppositely charged proteinaceous microgel; and
wherein the weight ratio of one or more polysaccharide-based nanofibrils to proteinaceous microgel is from about 0.1:1 to about 10:1;
wherein the % outer surface coverage of the microgel by the nanofibrils is from about 50% to about 99%,

wherein the % outer surface coverage of the microgel by the nanofibrils ($c/c_{sat}$) is calculated by the following equation:

$$\frac{3c}{c_{sat}} = -ln\left(\frac{\zeta_c - \zeta_{sat}}{\zeta_0 - \zeta_{sat}}\right)$$

wherein:

$\zeta_{sat}$ is the $\zeta$-potential when the microgels are saturated with polysaccharide-based nanofibrils;
$\zeta_0$ is the $\zeta$-potential of the proteinaceous microgel in absence of the polysaccharide-based nanofibrils;
$\zeta_c$ is the $\zeta$-potential of the formulation at polysaccharide-based nanofibril concentration $c$; and
$c_{sat}$ is the minimum amount of the polysaccharide-based nanofibrils required to completely cover the surface of the proteinaceous microgel.

9.  The use of the formulation or the formulation for use of claim 8, wherein the weight ratio of one or more polysaccharide-based nanofibrils to proteinaceous microgel is from about 0.2:1 to about 3:1.

10. The use of the formulation or the formulation for use of claim 8 or claim 9, wherein the proteinaceous microgel is a lactoferrin microgel.

11. The use of the formulation or the formulation for use of any of claims 8 to 10, wherein the one or more polysaccharide-based nanofibrils are κ-carrageenan nanofibrils.

12. The use of the formulation or the formulation for use of any of claims 8 to 11, wherein the one or more polysaccharide-based nanofibrils associated with the outer surface of the proteinaceous microgel result in an outer surface that has an overall negative charge.

13. The use of the formulation or the formulation for use of any of claims 8 to 12, wherein the formulation is a colloidosome, optionally wherein the colloidosome is no more than 1000 nm in diameter.

14. The use of the formulation or the formulation for use of any of claims 8 to 13, wherein the proteinaceous microgel has a pH of from about 3.0 to about 4.0, or of about 7.0.


**Patentansprüche**

1.  Verfahren zum Herstellen einer Formulierung, wobei das Verfahren Folgendes umfasst:

    (a) Auflösen eines proteinösen Materials in einer Pufferlösung und Erwärmen der resultierenden Lösung, um ein proteinöses Mikrogel oder ein thermofixiertes Gel zu bilden;
    (b) wenn Schritt (a) in einem thermofixierten Gel resultiert, Mischen des thermofixierten Gels mit der Pufferlösung und Homogenisieren, um ein proteinöses Mikrogel zu bilden, wobei das proteinöse Mikrogel ausgewählt ist aus der Gruppe bestehend aus: Lactoferrin, Lysozym, Gelatine, wie Gelatine Typ B, Milchprotein, Molkenprotein, Kasein, Kaseinat, Eiprotein, Albumin, wie Rinderserumalbumin, Gluten, Erbsenprotein, Reisprotein, Legumin, Maisprotein, Erdnussprotein und Kartoffelprotein, wobei das proteinöse Mikrogel einen Durchmesser von etwa 100 nm bis etwa 500 nm aufweist;
    (c) Herstellen einer Lösung aus einer oder mehreren Nanofibrillen auf Polysaccharidbasis durch: (i) Erwärmen einer Mischung aus einem oder mehreren Polysaccharidmaterialien und einer Pufferlösung, unter Scheren der Mischung, um die eine oder mehreren Nanofibrillen auf Polysaccharidbasis zu bilden, (ii) Abkühlen der resultierenden wässrigen Dispersion, die die Nanofibrillen umfasst; und
    (d) Zugeben des proteinösen Mikrogels aus Schritt (a) oder Schritt (b) zur Lösung einer oder mehrerer Nanofibrillen auf Polysaccharidbasis aus Schritt (c), um die Formulierung zu bilden, wobei die Nanofibrillen auf Polysaccharidbasis ausgewählt sind aus der Gruppe bestehend aus: κ-Carrageen, ι-Carrageen, λ-Carrageen, Agar, Agarose, Alginat, Pektin, Dextransulfat, Cellulose, Xanthangummi, Gellangummi und einem beliebigen negativ geladenen Polysaccharid; wobei die eine oder mehreren Nanofibrillen auf Polysaccharidbasis einen äquivalenten Durchmesser von etwa 1 nm bis etwa 100 nm und eine Länge von etwa 50 nm bis etwa 500 nm aufweisen,
    wobei das proteinöse Mikrogel positiv geladen ist und einen pH von etwa 3,0 bis etwa 7,0 aufweist und die eine oder mehreren auf Nanofibrillen auf Polysaccharidbasis negativ geladen sind;

wobei das Gewichtsverhältnis von einer oder mehreren Nanofibrillen auf Polysaccharidbasis zu proteinösem Mikrogel von etwa 0,1:1 bis etwa 10:1 beträgt;

wobei die resultierende Formulierung die eine oder mehreren Nanofibrillen auf Polysaccharidbasis assoziiert mit einer äußeren Oberfläche des proteinösen Mikrogels aufweist; und

wobei die Menge an Mikrogel, die Nanofibrillen zugegeben wird, so ausgewählt ist, dass die prozentuale äußere Oberflächenbedeckung des Mikrogels durch die Nanofibrillen etwa 50 % bis etwa 99 % beträgt,

wobei die prozentuale äußere Oberflächenbedeckung des Mikrogels durch die Nanofibrillen ( $c/c_{sat}$ ) berechnet wird durch die folgende Gleichung:

$$\frac{3c}{c_{sat}} = -ln\left(\frac{\zeta_c - \zeta_{sat}}{\zeta_0 - \zeta_{sat}}\right)$$

wobei:

$\zeta_{sat}$ das $\zeta$-Potential ist, wenn die Mikrogele mit Nanofibrillen auf Polysaccharidbasis gesättigt sind;
$\zeta_0$ das C-Potential des proteinösen Mikrogels in Abwesenheit der Nanofibrillen auf Polysaccharidbasis ist;
$\zeta_c$ das $\zeta$-Potential der Formulierung bei Nanofibrillenkonzentration $c$ auf Polysaccharidbasis ist; und
$c_{sat}$ die Mindestmenge an Nanofibrillen auf Polysaccharidbasis ist, die erforderlich ist, um die Oberfläche des proteinösen Mikrogels vollständig zu bedecken.

2. Verfahren nach Anspruch 1, wobei das Gewichtsverhältnis von einer oder mehreren Nanofibrillen auf Polysaccharidbasis zu proteinösem Mikrogel von etwa 0,2: 1 bis etwa 3:1 beträgt.

3. Verfahren nach einem vorhergehenden Anspruch, wobei das proteinöse Mikrogel ein Lactoferrinmikrogel ist.

4. Verfahren nach einem vorhergehenden Anspruch, wobei die eine oder mehreren Nanofibrillen auf Polysaccharidbasis $\kappa$-Carrageen-Nanofibrillen sind.

5. Verfahren nach einem vorhergehenden Anspruch, wobei die resultierende Formulierung ein Kolloidosom ist, wobei das Kolloidosom optional nicht mehr als 1000 nm im Durchmesser ist.

6. Verfahren nach einem vorhergehenden Anspruch, wobei das proteinöse Mikrogel einen pH von etwa 3,0 bis etwa 4,0 oder von etwa 7,0 aufweist.

7. Formulierung, die durch das Verfahren nach einem vorhergehenden Anspruch erhalten werden kann oder erhalten wird.

8. Verwendung einer Formulierung als ein Schmiermittel-Lebensmittelzusatzstoff; oder einer Formulierung zur Verwendung bei der Behandlung einer Erkrankung oder eines Leidens, ausgewählt aus: Mundtrockenheit, Xerostomie oder Dysphagie;

wobei der trockene Mund optional verursacht wird durch: Speicheldrüsenerkrankungen und -störungen, chronische entzündliche Autoimmunerkrankungen, Sjögren-Syndrom, endokrine Erkrankungen, Diabetes, neurologische Erkrankungen und -störungen, psychogene Erkrankungen, Angst, Nervosität, HIV/AIDS und Polypharmazie;

wobei die Formulierung Folgendes umfasst:

(i) ein proteinöses Mikrogel, ausgewählt aus der Gruppe bestehend aus: Lactoferrin, Lysozym, Gelatine, wie Gelatine Typ B, Milchprotein, Molkenprotein, Kasein, Kaseinat, Eiprotein, Albumin, wie Rinderserumalbumin, Gluten, Erbsenprotein, Reisprotein, Legumin, Maisprotein, Erdnussprotein und Kartoffelprotein; wobei das proteinöse Mikrogel einen Durchmesser von etwa 100 nm bis etwa 500 nm aufweist; und

(ii) eine oder mehrere Nanofibrillen auf Polysaccharidbasis, ausgewählt aus der Gruppe bestehend aus: $\kappa$-Carrageen, $\iota$-Carrageen, $\lambda$-Carrageen, Agar, Agarose, Alginat, Pektin, Dextransulfat, Cellulose, Xanthangummi, Gellangummi und einem beliebigen negativ geladenen Polysaccharid; wobei die eine oder mehreren Nanofibrillen auf Polysaccharidbasis einen äquivalenten Durchmesser von etwa 1 nm bis etwa 100 nm und eine Länge von etwa 50 nm bis etwa 500 nm aufweisen;

wobei das proteinöse Mikrogel positiv geladen ist und einen pH von etwa 3,0 bis etwa 7,0 aufweist und die eine oder mehreren auf Nanofibrillen auf Polysaccharidbasis negativ geladen ist;

wobei die eine oder mehreren Nanofibrillen auf Polysaccharidbasis mit einer äußeren Oberfläche des entgegengesetzt geladenen proteinösen Mikrogels assoziiert sind; und

wobei das Gewichtsverhältnis von einer oder mehreren Nanofibrillen auf Polysaccharidbasis zu proteinösem Mikrogel von etwa 0,1:1 bis etwa 10:1 beträgt;

wobei die prozentuale äußere Oberflächenbedeckung des Mikrogels durch die Nanofibrillen etwa 50 % bis etwa 99 % beträgt,

wobei die prozentuale äußere Oberflächenbedeckung des Mikrogels durch die Nanofibrillen ($c/c_{sat}$) berechnet wird durch die folgende Gleichung:

$$\frac{3c}{c_{sat}} = -ln\left(\frac{\zeta_c - \zeta_{sat}}{\zeta_0 - \zeta_{sat}}\right)$$

wobei:

$\zeta_{sat}$ das $\zeta$-Potential ist, wenn die Mikrogele mit Nanofibrillen auf Polysaccharidbasis gesättigt sind;
$\zeta_0$ das $\zeta$-Potential des proteinösen Mikrogels in Abwesenheit der Nanofibrillen auf Polysaccharidbasis ist;
$\zeta_c$ das $\zeta$-Potential der Formulierung bei Nanofibrillenkonzentration $c$ auf Polysaccharidbasis ist; und
$c_{sat}$ die Mindestmenge an Nanofibrillen auf Polysaccharidbasis ist, die erforderlich ist, um die Oberfläche des proteinösen Mikrogels vollständig zu bedecken.

9. Verwendung der Formulierung oder der Formulierung zur Verwendung nach Anspruch 8, wobei das Gewichtsverhältnis von einer oder mehreren Nanofibrillen auf Polysaccharidbasis zu proteinösem Mikrogel von etwa 0,2:1 bis etwa 3:1 beträgt.

10. Verwendung der Formulierung oder der Formulierung zur Verwendung nach Anspruch 8 oder Anspruch 9, wobei das proteinöse Mikrogel ein Lactoferrinmikrogel ist.

11. Verwendung der Formulierung oder der Formulierung zur Verwendung nach einem der Ansprüche 8 bis 10, wobei die eine oder mehreren Nanofibrillen auf Polysaccharidbasis κ-Carrageen-Nanofibrillen sind.

12. Verwendung der Formulierung oder der Formulierung zur Verwendung nach einem der Ansprüche 8 bis 11, wobei die eine oder mehreren Nanofibrillen auf Polysaccharidbasis, die mit der äußeren Oberfläche des proteinösen Mikrogels assoziiert sind, in einer äußeren Oberfläche resultieren, die eine insgesamt negative Ladung aufweist.

13. Verwendung der Formulierung oder der Formulierung zur Verwendung nach einem der Ansprüche 8 bis 12, wobei die Formulierung ein Kolloidosom ist, wobei das Kolloidosom optional nicht mehr als 1000 nm im Durchmesser ist.

14. Verwendung der Formulierung oder der Formulierung zur Verwendung nach einem der Ansprüche 8 bis 13, wobei das proteinöse Mikrogel einen pH von etwa 3,0 bis etwa 4,0 oder von etwa 7,0 aufweist.

**Revendications**

1. Procédé permettant la préparation d'une formulation, le procédé comprenant :

(a) la dissolution d'un matériau protéique dans une solution tampon et le chauffage de la solution résultante pour former un microgel protéique ou un gel thermodurci ;
(b) lorsque l'étape (a) conduit à un gel thermodurci, le mélange du gel thermodurci avec la solution tampon et l'homogénéisation pour former un microgel protéique, ledit microgel protéique étant choisi dans le groupe constitué par : la lactoferrine, le lysozyme, la gélatine, telle que la gélatine de type B, la protéine de lait, la protéine de lactosérum, la caséine, le caséinate, la protéine d'œuf, l'albumine, telle que l'albumine de sérum bovin, le gluten, la protéine de pois, la protéine de riz, la légumine, la protéine de maïs, la protéine d'arachide et la protéine de pomme de terre, ledit microgel protéique présentant un diamètre d'environ 100 nm à environ 500 nm ;

(c) la préparation d'une solution d'une ou plusieurs nanofibrilles à base de polysaccharides par : (i) chauffage d'un mélange d'un ou plusieurs matériaux polysaccharidiques et d'une solution tampon tout en cisaillant le mélange pour former lesdites une ou plusieurs nanofibrilles à base de polysaccharides, (ii) refroidissement de la dispersion aqueuse résultante comprenant les nanofibrilles ; et

(d) l'ajout du microgel protéique de l'étape (a) ou de l'étape (b) à la solution d'une ou plusieurs nanofibrilles à base de polysaccharides de l'étape (c) pour former la formulation, lesdites nanofibrilles à base de polysaccharides étant choisies dans le groupe constitué par : la $\kappa$-carraghénane, la $\iota$-carraghénane, la $\lambda$-carraghénane, la gélose, l'agarose, l'alginate, la pectine, le sulfate de dextrane, la cellulose, la gomme de xanthane, la gomme de gellane et tout polysaccharide chargé négativement ; lesdites une ou plusieurs nanofibrilles à base de polysaccharides présentant un diamètre équivalent d'environ 1 nm à environ 100 nm et une longueur d'environ 50 nm à environ 500 nm,

ledit microgel protéique étant chargé positivement présentant un pH d'environ 3,0 à environ 7,0, et lesdites une ou plusieurs nanofibrilles à base de polysaccharides étant chargées négativement ;

ledit rapport pondéral d'une ou de plusieurs nanofibrilles à base de polysaccharides au microgel protéique étant d'environ 0,1:1 à environ 10:1 ;

ladite formulation résultante comportant lesdites une ou plusieurs nanofibrilles à base de polysaccharides associées à une surface externe du microgel protéique ; et

ladite quantité de microgel qui est ajoutée aux nanofibrilles étant choisie de sorte que le % de couverture de la surface extérieure du microgel par les nanofibrilles soit d'environ 50 % à environ 99 %,

ledit % de couverture de la surface externe du microgel par les nanofibrilles ( $c/c_{sat}$ ) étant calculé par l'équation suivante :

$$\frac{3c}{c_{sat}} = -ln\left(\frac{\zeta_c - \zeta_{sat}}{\zeta_0 - \zeta_{sat}}\right)$$

dans laquelle :

$\zeta_{sat}$ représente le $\zeta$-potentiel lorsque les microgels sont saturés de nanofibrilles à base de polysaccharides ;
$\zeta_0$ représente le $\zeta$-potentiel du microgel protéique en l'absence des nanofibrilles à base de polysaccharides ;
$\zeta_c$ représente le $\zeta$-potentiel de la formulation à la concentration de nanofibrilles à base de polysaccharides $c$ ; et
$c_{sat}$ représente la quantité minimale de nanofibrilles à base de polysaccharides nécessaire pour recouvrir complètement la surface du microgel protéique.

2. Procédé selon la revendication 1, ledit rapport pondéral d'une ou de plusieurs nanofibrilles à base de polysaccharides au microgel protéique étant d'environ 0,2:1 à environ 3:1.

3. Procédé selon l'une quelconque des revendications précédentes, ledit microgel protéique étant un microgel de lactoferrine.

4. Procédé selon l'une quelconque des revendications précédentes, lesdites une ou plusieurs nanofibrilles à base de polysaccharides étant des nanofibrilles de $\kappa$-carraghénane.

5. Procédé selon l'une quelconque des revendications précédentes, ladite formulation résultante étant un colloïdosome, éventuellement ledit colloïdosome ne dépassant pas 1000 nm de diamètre.

6. Procédé selon l'une quelconque des revendications précédentes, ledit microgel protéique présentant un pH d'environ 3,0 à environ 4,0, ou d'environ 7,0.

7. Formulation obtenue ou pouvant être obtenue par le procédé selon l'une quelconque des revendications précédentes.

8. Utilisation d'une formulation comme additif alimentaire lubrifiant ; ou formulation destinée à être utilisée dans le traitement d'une maladie ou d'une affection choisie parmi : la sécheresse buccale, la xérostomie ou la dysphagie ;

éventuellement ladite bouche sèche étant causée par : les maladies et troubles des glandes salivaires, les maladies auto-immunes inflammatoires chroniques, le syndrome de Sjögren, les maladies endocriniennes, le diabète, les maladies et troubles neurologiques, les maladies psychogènes, l'anxiété, la nervosité, le VIH/sida et la polypharmacie ;
ladite formulation comprenant :

(i) un microgel protéique, choisi dans le groupe constitué par : la lactoferrine, le lysozyme, la gélatine, telle que la gélatine de type B, la protéine de lait, la protéine de lactosérum, la caséine, le caséinate, la protéine d'œuf, l'albumine, telle que l'albumine de sérum bovin, le gluten, la protéine de pois, la protéine de riz, la légumine, la protéine de maïs, la protéine d'arachide et la protéine de pomme de terre ; ledit microgel protéique présentant un diamètre d'environ 100 nm à environ 500 nm ; et
(ii) une ou plusieurs nanofibrilles à base de polysaccharides, choisies dans le groupe constitué par : la κ-carraghénane, la ι-carraghénane, la λ-carraghénane, la gélose, l'agarose, l'alginate, la pectine, le sulfate de dextrane, la cellulose, la gomme de xanthane, la gomme de gellane et tout polysaccharide chargé négativement ; lesdites une ou plusieurs nanofibrilles à base de polysaccharides présentant un diamètre équivalent d'environ 1 nm à environ 100 nm et une longueur d'environ 50 nm à environ 500 nm ;

ledit microgel protéique étant chargé positivement présentant un pH d'environ 3,0 à environ 7,0, et lesdites une ou plusieurs nanofibrilles à base de polysaccharides étant chargées négativement ;
lesdites une ou plusieurs nanofibrilles à base de polysaccharides étant associées à une surface externe du microgel protéique chargé de manière opposée ; et
ledit rapport pondéral d'une ou de plusieurs nanofibrilles à base de polysaccharides au microgel protéique étant d'environ 0,1:1 à environ 10:1 ;
ledit % de couverture de la surface externe du microgel par les nanofibrilles étant d'environ 50 % à environ 99 %,
ledit % de couverture de la surface externe du microgel par les nanofibrilles ($c/c_{sat}$) étant calculé par l'équation suivante :

$$\frac{3c}{c_{sat}} = -ln\left(\frac{\zeta_c - \zeta_{sat}}{\zeta_0 - \zeta_{sat}}\right)$$

dans laquelle :

$\zeta_{sat}$ représente le $\zeta$-potentiel lorsque les microgels sont saturés de nanofibrilles à base de polysaccharides ;
$\zeta_0$ représente le $\zeta$-potentiel du microgel protéique en l'absence des nanofibrilles à base de polysaccharides ;
$\zeta_c$ représente le $\zeta$-potentiel de la formulation à la concentration de nanofibrilles à base de polysaccharides c ; et
$c_{sat}$ représente la quantité minimale de nanofibrilles à base de polysaccharides nécessaire pour recouvrir complètement la surface du microgel protéique.

9. Utilisation de la formulation ou formulation destinée à être utilisée selon la revendication 8, ledit rapport pondéral d'une ou de plusieurs nanofibrilles à base de polysaccharides au microgel protéique étant d'environ 0,2:1 à environ 3:1.

10. Utilisation de la formulation ou formulation destinée à être utilisée selon la revendication 8 ou la revendication 9, ledit microgel protéique étant un microgel de lactoferrine.

11. Utilisation de la formulation ou formulation destinée à être utilisée selon l'une quelconque des revendications 8 à 10, lesdites une ou plusieurs nanofibrilles à base de polysaccharides étant des nanofibrilles de κ-carraghénane.

12. Utilisation de la formulation ou formulation destinée à être utilisée selon l'une quelconque des revendications 8 à 11, lesdites une ou plusieurs nanofibrilles à base de polysaccharides associées à la surface externe du microgel protéique conduisant à une surface externe qui a une charge négative globale.

13. Utilisation de la formulation ou formulation destinée à être utilisée selon l'une quelconque des revendications 8 à 12, ladite formulation étant un colloïdosome, éventuellement ledit colloïdosome ne dépassant pas 1000 nm de diamètre.

14. Utilisation de la formulation ou formulation destinée à être utilisée selon l'une quelconque des revendications 8 à 13, ledit microgel protéique présentant un pH d'environ 3,0 à environ 4,0, ou d'environ 7,0.

**FIG 1**

**FIG 2**

FIG 3

FIG 4

FIG 5

FIG 6a

FIG 6b

FIG 7

FIG 8

FIG 9

FIG 10

FIG 11

FIG 12

FIG 13

FIG 14

FIG 15

FIG 16

**FIG 17**

**FIG 18**

FIG 19

FIG 20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018064512 B
- JP 2016063832 B
- CN 108578357


**Non-patent literature cited in the description**

- **FURNESS S** ; **WORTHINGTON HV** ; **BRYAN G** ; **BIRCHENOUGH S** ; **MCMILLAN R**. Interventions for the management of dry mouth: topical therapies. *Cochrane Database of Systematic Reviews*, 2011
- **HOPCRAFT M** ; **TAN C**. *Xerostomia: an update for clinicians*, 2010, vol. 55, 238-44
- **VILLA A** ; **ABATI S**. *Risk factors and symptoms associated with xerostomia: a cross-sectional study*, 2011, vol. 56, 290-5
- **PORTER SR** ; **SCULLY C** ; **HEGARTY AM**. An update of the etiology and management of xerostomia. *Oral Surgery, Oral Medicine, Oral Pathology, Oral Radiology, and Endodontology*, 2004, vol. 97, 28-46
- **THELIN W** ; **BRENNAN M** ; **LOCKHART P** ; **SINGH M** ; **FOX P** ; **PAPAS A et al.** *The oral mucosa as a therapeutic target for xerostomia*, 2008, vol. 14, 683-9
- **NÄRHI TO** ; **MEURMAN JH** ; **AINAMO A**. Xerostomia and Hyposalivation. *Drugs & Aging.*, 1999, vol. 15, 103-16
- **HAN P** ; **SUAREZ-DURALL P** ; **MULLIGAN R**. Dry mouth: A critical topic for older adult patients. *Journal of Prosthodontic Research*, 2015, vol. 59, 6-19
- **NARHI TO** ; **MEURMAN JH** ; **AINAMO A**. Xerostomia and hyposalivation: causes, consequences and treatment in the elderly. *Drugs Aging.*, 1999, vol. 15, 103-16
- **ANDABLO-REYES E** ; **YERANI D** ; **FU M** ; **LIAMAS E** ; **CONNELL S** ; **TORRES O et al.** Microgels as viscosity modifiers influence lubrication performance of continuum. *Soft Matter.*, 2019, vol. 15, 9614-24
- **SARKAR A** ; **KANTI F** ; **GULOTTA A** ; **MURRAY BS** ; **ZHANG S**. Aqueous lubrication, structure and rheological properties of whey protein microgel particles. *Langmuir*, 2017, vol. 33, 14699-708
- **TORRES O** ; **ANDABLO-REYES E** ; **MURRAY BS** ; **SARKAR A**. Emulsion microgel particles as high-performance bio-lubricants. *ACS Applied Materials & Interfaces.*, 2018, vol. 10, 26893-905
- **XU F** ; **LIAMAS E** ; **BRYANT M** ; **ADEDEJI AF** ; **ANDABLO-REYES E** ; **CASTRONOVO M et al.** A self-assembled binary protein model explains high-performance salivary lubrication from macro to nanoscale. *Advanced Materials Interfaces.*, 2020, vol. 7, 1901549
- **HAN, P.** ; **P. SUAREZ-DURALL** ; **R. MULLIGAN**. Dry mouth: a critical topic for older adult patients. *J Prosthodont Res*, 2015, vol. 59 (1), 6-19
- **OLIVARES M L**. Soft lubrication characteristics of microparticulated whey proteins used as fat replacers in dairy systems. *Journal of Food Engineering*, 13 October 2018, vol. 245, 157-165
- **ANWESHA SARKAR et al.** Lubrication of soft oral surfaces. *Current Opinion in Colloid & Interfacial Science*, 01 February 2019, vol. 39, 61-75
- **ANDREA ARAIZA CALAHORRA**. Pickering emulsion-based encapsulation strategies for delivery of curcumin. The University of Leeds, 01 March 2020, 1-263
- **ANGES TEO** ; **SUNG JE LEE** ; **KELVIN K. T. GOH**. *Food Structure*, 2017, vol. 14, 60-67
- **ANWESHA SARKAR** ; **KELVIN K.T. GOH** ; **HARJINDER SINGH**. *Food Hydrocolloids*, 2009, vol. 23, 1270-1278
- **S. PALLANDRE** ; **E. A. DECKER** ; **D. J. MCCLEMENTS**. *Journal of Food Science*, 2007, vol. 72, E518-E524
- **DEMET GUZEY** ; **DAVID JULIAN MCCLEMENTS**. *J. Agric. Food Chem.*, 2007, vol. 55, 475-485